# EUROPEAN PATENT APPLICATION

(11) **EP 2 357 478 A2**
(43) Date of publication of application: **17.08.2011**
(21) Application number: 10186268.8
(22) Date of filing: 30.11.2001
(51) Int. Cl.: G01N 33/68, G01N 33/564

(54) **Diagnosis of disease**

(30) Priority: 30.11.2000 GB 0029225; 07.12.2000 GB 0029879
(62) Divisional of application: 01998835.1
(71) Applicant: Crawford Healthcare Holdings Limited, Goostrey Cheshire CW4 8JP (GB)
(72) Inventor: Tazi-Ahnini, Rachid, Sheffield, Yorkshire S10 2RX (GB); Duff, Gordon, Sheffield,Yorkshire S10 2RX (GB); Cork, Michael, Sheffield, Yorkshire S10 2RX (GB); Ward, Simon, Sheffield, Yorkshire S10 2RX (GB); Bavik, Claes, Sheffield, Yorkshire S10 2RX (GB)
(74) Representative: Finnie, Isobel Lara

(57) **Abstract**

We disclose a method of diagnosis of a disease, or susceptibility to a disease associated with abnormal cell-cell adhesion between epithelial cells, the method comprising detection of a mutation in a nucleic acid encoding an adhesion protein, a protease, or a protease inhibitor of an individual.

## Description

### FIELD

This invention relates to the diagnosis of diseases. More particularly, the invention relates to the diagnosis of epidermal or skin diseases, and agents for such diagnosis.

### BACKGROUND

The skin is a barrier that retains water within the body and prevents the penetration of environmental agents into the body. The barrier function of the skin is therefore essential to the maintenance of the internal homeostasis (Cork 1997). The epidermis is composed of layers of closely packed keratinocytes that are formed by division in the stratum basale (or germinative layer). As the keratinocytes move up through the prickle and granular layers, they differentiate and a rigid internal structure of keratin, microfilaments and microtubules is formed. The stratum corneum (horny layer) is composed of layers of flattened dead cells that have lost their nucleus, between which is a complex mixture of lipid and proteins.

The epidermal barrier is located in the stratum corneum and is dependent on strong adhesion between corneocytes (Egelrud, 2000). This adhesion is mediated by corneodesmosomes (Menton and Elisen, 1971 Chapman and Walsh, 1990; North et al, 1999). Corneodesmosin is a glycoprotein of corneodesmosomes.

The other component of the epidermal barrier is the lamellar lipids. The lipid is secreted into lamellar bodies in the stratum granulosum (Elias 1993). At the interface between the stratum granulosum and stratum corneum, the lipids are extracted from the granular cells into the intercorneoctye space. The lipids are then organised into highly organised multimellar bilayer structures (Landmann 1988). The stratum corneum can be visualised rather like a brick wall with the corneocytes forming the bricks and the lamellar lipids the mortar (Elias 1983). Corneocytes contain a water retaining substance, natural moisturising factor (NMF), which retains water within them. The high water content causes the corneocytes to swell, preventing the formation of fissures and cracks between them. The pliability and elasticity of the skin is directly related to its water content. Normal healthy stratum corneum has comparatively high water content.

Thus, the stratum corneum is a barrier that is continually being replaced by proliferation and differentiation ofkeratinocytes in the viable epidermis. In order to maintain a constant stratum corneum thickness at a given body site superficial parts of the stratum corneum must be continuously shed in the process of desquamation at a rate that balances their production. Impaired desquamation of corneocytes is characteristic of a number of diseases such as psoriasis, acne vulgaris, ichiosis and keratinosis pilaris, among others. In psoriasis, impaired desquamation of corneocytes causes an increased thickness of the stratum corneum and the barrier is usually enhanced.

### SUMMARY

We demonstrate that proteolysis of corneodesmosomal proteins is a key process involved in desquamation.

Accordingly, we have discovered that increased, impaired or otherwise abnormal proteolysis of corneodesmosomal proteins is involved in several skin disorders. In particular, we have found out that increased proteolysis of adhesion proteins is involved in diseases characterised by impaired barrier function, and decreased proteolysis of adhesion proteins is involved in diseases characterised by impaired desquamation of corneocytes. We further demonstrate that mutations in genes encoding adhesion proteins result in reduced adhesion between epithelial cells.

According to a first aspect of the present invention, we provide, a method of diagnosis of a Group I disease or susceptibility to a Group I disease in an individual, the method comprising detecting a presence or absence of a polymorphism in an adhesion protein, polypeptide, or a nucleic acid encoding such, in which the polymorphism is associated with a Group I disease.

Preferably, the adhesion protein is selected from the group consisting of adhesion proteins shown in Tables D2.1 and 5.1, preferably comeodesmosin, desmoglein I, desmoglein 3, plakoglobin, desmoplakin, desmocollin I and envoplakin,a serine rich protein, preferably a small proline-rich protein (SPRR), SPRR2A, SPRRIB, SPRK, SPRR2E, SPRR2F, SPRR2B, SPRR2D, SPRR2C, SPRR2G, SPRRIA, SPRR3, SPRR4, involucrin, or loricrin,

Preferably, the Group I disease is selected from the group consisting of: atopic eczema, sebarrhoeic eczema, irritant contact dermatitis, allergic contact dermatitis, lung atopic asthma, post viral asthma, branchial hyper-reactivity, chronic obstruction pulmonary disease, Crohn's disease, ulcerative colitis, coeliac disease, peptic ulceration, impetigo, viral warts, Molluslum Contagiosum, bacterial meningitis, viral meningitis, peptic ulceration associated with penetration of Helicobacteria pylori, skin melanoma, squamous cell carcinoma, basal cell carcinoma, cutaneous lymphoma, a skin cancer, a malignancy of the gastrointestinal tract and a malignancy of the lung.

We provide, according to a second aspect of the present invention, a method of diagnosis of a Group I disease or susceptibility to a Group I disease in an individual, the method comprising detecting the presence, absence or a modulated level of an adhesion protein, or a fragment thereof, in an individual.

Preferably, the Group I disease is selected from the group consisting of: atopic eczema, sebarrhoeic eczema, irritant contact dermatitis, allergic contact dermatitis, lung atopic asthma, post viral asthma, branchial hyper-reactivity, chronic obstruction pulmonary disease, Crohn's disease, ulcerative colitis, coeliac disease, peptic ulceration, impetigo, viral warts, Molluscum Contagiosum, bacterial meningitis, viral meningitis, peptic ulceration associated with penetration of Helicobacteria pylori, skin melanoma, squamous cell carcinoma, basal cell carcinoma, cutaneous lymphoma, a skin cancer, a malignancy of the gastrointestinal tract and a malignancy of the lung.

Preferably, the Group I disease is eczema, preferably atopic eczema, or dermatitis, preferably dermatitis herpetiformis.

There is provided, in accordance with a third aspect of the present invention, a monoclonal or polyclonal antibody capable of specifically reacting with an adhesion protein, preferably a disease associated form of an adhesion protein.

### DETAILED DESCRIPTION

We demonstrate that regulated proteolysis of adhesion proteins is important for a healthy skin. We show that an underlying cause of various skin diseases is the breakdown in regulation of proteolysis of adhesion proteins, leading to an increased, decreased or otherwise abnormal adhesion between corneocytes. Such abnormal proteolysis may have various causes, including mutations in adhesion protein genes, mutations in proteases which act on adhesion proteins, and/or in protease inhibitors which act on such proteases and regulate their activity.

We show that treatment and prevention of such diseases may be achieved by modulating the proteolysis of adhesion proteins. We also demonstrate that detection of such mutations in adhesion proteins, proteases and/or protease inhibitors may be used to diagnose various skin diseases and/or susceptibility to such diseases.

Where reference is made to "diagnosis" of a disease, this should be taken to include both diagnosis of the disease itself, as well as susceptibility to the disease. The methods of diagnosis disclosed here may also be employed as methods of providing indications useful in the diagnosis of diseases.

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 2d ed. (1989) Cold Spring Harbor Laboratory Press, Cold Spring Harbor, N.Y. and Ausubel et al., Short Protocols in Molecular Biology (1999) 4th Ed, John Wiley & Sons, Inc. which are incorporated herein by reference), chemical methods pharmaceutical formulations and delivery and treatment of patients.

### EPIDERMAL BARRIER FUNCTION

The methods and compositions described here are primarily concerned with the diagnosis of diseases associated with or characterised by abnormal epidermal barrier function.

The skin is a barrier that retains water within the body and prevents the penetration of environmental agents into the body. This ability to resist penetration is essential to the maintenance of the internal homeostasis (Cork 1997). The epidermis is composed of layers of closely packed keratinocytes that are formed by division in the stratum basale (or germinative layer). As the keratinocytes move up through the prickle and granular layers, they differentiate and a rigid internal structure of keratin, micro filaments and microtubules is formed. Within the epidermis, the barrier function is typically performed by the stratum corneum and is dependent on strong adhesion between corneocytes (Egelrud, 2000) mediated by corneodesmosomes (Menton and Elisen, 1971, Chapman and Walsh, 1990; North et al, 1999).

The stratum corneum (horny layer) is composed of layers of flattened dead cells that have lost their nucleus, between which is a complex mixture of lipid and proteins. The stratum corneum is a barrier that is continually being replaced by proliferation and differentiation of keratinocytes in the viable epidermis. In order to maintain a constant stratum corneum thickness at a given body site superficial parts of the stratum corneum must be continuously shed in the process of desquamation at a rate that balances their production.

The "barrier function" of the epidermis, or the "epidermal barrier function", as the terms are used in this document, is therefore intended to refer to the ability of an epidermal layer to resist the penetration of an external agent. Accordingly, the methods and compositions act by modulating the epidermal barrier function of an individual. In particular, the methods and compositions described here may be used to diagnose diseases in which the epidermal barrier in individuals suffering from such diseases is modulated, i.e., enhanced or weakened, as compared to the epidermal barrier in normal individuals.

Measurement of barrier function may be done in various ways. For example, a Franz chamber and cadaver system (also known as a Franz chamber penetration system) may be used. This system measures barrier function by measuring the substances passing through it, and has the advantage in that it is a robust, quick and easy system.

### Impaired Barrier Function

Impaired barrier function is a characteristic of Group I diseases, described in detail below.

An epidermis has an impaired barrier function when it is more permeable to an external agent than a normal, healthy epidermis from the same or another individual. Thus, an epidermis with impaired barrier function allows more penetration of an external agent than otherwise. Preferably, an epidermis with impaired barrier function is 20%, 40%, 60%, 80% or 100% or more permeable to an external agent than a normal epidermis. Preferably, a molecule which is substantially unable to cross a normal epidermal barrier is able to cross the barrier of an epidermis with impaired barrier function.

Increase in penetration or permeability is preferably reflected by increase of mass of agent or drug, activity of an agent or drug (such as a relevant chemical, biological or enzymatic activity) which is capable of passing through the epidermal layer. Thus, an epidermis with impaired barrier function will therefore preferably enable penetration of 5%, 10%, 20%, 30%, 40%, 50%, 60%, 70%, 80% 90% or 100% or more agent or drug than a normal epidermis.

Increase in permeability or penetration may also be reflected by an increased ratio of molecules which pass through compared to those which are retarded, or alternatively, as compared to those which are applied. This ratio, N, may be increased by 5%, 10%, 20%, 30%, 40%, 50%, 60%, 70%, 80% 90% or 100% or more with the methods and compositions as described here.

Measurement of epidermal barrier function may be done in various ways. Similarly, the level or degree of penetration of an agent or composition can be determined by techniques known to those of skill in the art. For example, radiolabeling of an active compound or a tracer compound, followed by measurement of the amount of radiolabelled compound absorbed by the skin enables one of skill in the art to determine levels of the composition absorbed using any of several methods of determining skin penetration of the test compound. Measurement of the amount of radiolabelled compound which crosses the skin layer may also be made.

In a preferred embodiment, a Franz chamber and cadaver system (also known as a Franz chamber penetration system) is used to measure penetration of an agent. This system measures barrier function by allowing the measurement of an amount of radiolballed compoud which passes through a piece of skin to a receptable fluid. The Franz chamber has the advantage in that it is a robust, quick and easy system and is described in more detail below.

### Enhanced Barrier Function

An epidermis which has increased barrier function is less permeable to an external agent than a normal, healthy epidermis from the same or other individual.

### GROUP I DISEASES: ASSOCIATED WITH DECREASED CELL-CELL ADHESION

In one embodiment, the methods disclosed here are suitable for diagnosis of diseases associated with decreased cell-cell adhesion, in particular of epithelial cells, in particular corneocytes. Such diseases are referred to in this document as "Group 1 diseases", and are therefore diseases of impaired or reduced barrier function.

Within the Group 1 diseases, three sub-groups may be identified. In each group the impaired barrier function is permitting the penetration of a xenobiotic through an epithelial barrier. Once the xenobiotic has penetrated through the epithelial barrier, it interacts with the host's cells to produce the disease phenotype.

A first subgroup of Group 1 diseases (Subgroup 1.1) is characterised by an impaired barrier that permits the penetration of an irritant, allergen or other xenobiotic. These xenobiotics interact with the body's immune system to produce an abnormal inflammatory response, which in turn causes tissue destruction. The immune response to the xenobiotic may be enhanced as the result of an associated genetic predisposition. Examples of such diseases affecting the skin include atopic eczema, sebarrhoeic eczema, irritant contact dermatitis, and allergic contact dermatitis. Examples of such diseass affecting the lung include: atopic asthma, post viral asthma/branchial hyper-reactivity, and chronic obstruction pulmonary disease. Examples of such diseases affecting the bowel include: Crohns disease, ulcerative colitis, coeliac disease, and peptic ulceration.

A second subgroup of Group 1 diseases (Subgroup 1.2) is characterised by an impaired barrier that permits the penetration of bacteria, virus, other micro-organisms or micro-organism products e.g. superantigenic exotoxin. The micro-organism and/or the micro-organism product then leads to a disease process. Examples of such diseases include atopic eczema, contact dermatits, impetigo, viral warts, Molluslum Molluscum Contagiosum, meningitis (bacterial and viral), and peptic ulceration caused by or associated with penetration of Helicobacteria pylori.

A third subgroup of Group 1 diseases (Subgroup 1.3) is characterised by an impaired barrier that permits the penetration of a carcinogen. The carcinogen can thereby gain access to the epithelial stem cell population and can induce transformation. The carcinogen may act as a co-carcinogen with other environmental agents e.g. ultraviolet radiation. Examples of such diseases affecting the skin include melanoma, squamous cell carcinoma, basal cell carcinoma, cutaneous lymphoma, and other skin cancers. Examples of such diseases affecting the bowel include malignancies of the entire gastrointestinal tract. Lung malignancies are examples of diseases affecting the lung.

### Treatment of Group I Diseases

According to the methods and compositions described here, Group 1 diseases result from a decreased adhesion between epithelial cells, for example corneocytes in the skin. This can arise as a result of changes in the expression, activity and/or breakdown of adhesion proteins which modulate or are responsible for the adhesion between epithelial cells, for example, corneodesmosin, changes in the expression, activity and/or breakdown of proteases which break down the adhesion proteins, and/or changes in the expression, activity and/or breakdown of inhibitors of proteases which break down the adhesion proteins. We have discovered that a change in any, some or all of the above may result in decreased adhesion between epithelial cells such as corneocytes.

As an example, an adhesion protein whose structure is changed as a result of a genetic mutation may have reduced adhesion activity and/or be more vulnerable to degradation by proteolytic enzymes (proteases). Accordingly, therefore, treatment of Group 1 diseases may be affected by increasing the expression of an adhesion protein, for example, comeodesmosin. Alternatively or in conjunction treatment may be effected by enhancing the adhesion activity and/or protease resistance of the adhesion protein. Thus, the adhesion protein responsible for reduced cell-cell adhesion may be replaced or supplemented with another adhesion protein, or a variant of the first adhesion protein with enhanced adhesion activity and/or protease resistance.

Furthermore, an increase in the quantity, activity and/or bio-availability of proteases may cause increased break down of adhesion proteins. Accordingly, therefore, a Group 1 disease may be treated by reducing the expression and/or activity of a protease responsible for breaking down an adhesion protein. For example, the transcription and/or translation of such a protease may be down-regulated as a means to treat Group 1 diseases. Thus, the expression of proteases such SCCE and SCTE may be decreased at the transcriptional and/or translational level to treat a Group 1 disease.

Impaired barrier function as a result of reduced cell-cell adhesion may also happen where there is a decreased quantity, activity and/or bio-availability of an inhibitor of a protease which breaks down an adhesion protein. Thus, a combination of any, some or all of the above changes may result in a greater decrease in cell-cell adhesion and impaired barrier function.

### Diagnosis of Group I Diseases

Mutations in adhesion proteins, proteases and/or protease inhibitors are associated with Group I diseases. Any of these mutations may be detected in the relevant gene, nucleic acid or polypeptide in order to diagnose a Group I disease.

### GROUP II DISEASES: ASSOCIATED WITH INCREASED CELL-CELL ADHESION

Diseases characterised by increased cell-cell adhesion of epithelial cells, in particular corneocytes are referred to in this document as "Group 2 diseases". Group 2 diseases are therefore diseases of increased or enhanced barrier function.

### Group II Diseases

Examples of Group 2 diseases include psoriasis, the ichthyoses, acne vulgaris and keratoses pilaris.

The Group 2 diseases may result from increased adhesion between epithelial, for example corneocytes in the skin. Increased adhesion results in an increased thickness of the stratum corneum. For example, in psoriasis and the ichthyoses, there is a generalised thickening of the stratum corneum. In acne there is a localised focal thickening of the stratum comeum at the entrance to the pilosebaceous duct.

Increased adhesion can arise as a result of changes in the expression, activity and/or breakdown of adhesion proteins which modulate or are responsible for the adhesion between epithelial cells, for example, corneodesmosin, changes in the expression, activity and/or breakdown of proteases which break down the adhesion proteins, and/or changes in the expression, activity and/or breakdown of inhibitors of proteases which break down the adhesion proteins. We have discovered that a change in any, some or all of the above may result in increased adhesion between epithelial cells such as corneocytes.

As an example, an adhesion protein whose structure is changed as a result of a genetic mutation may be more adhesive and/or less vulnerable to degradation by proteolytic enzymes (proteases). Accordingly, the methods and compositions described here may be used to treat Group 2 diseases by down-regulating the activity and/or expression of a mutant adhesion protein with enhanced adhesion activity. Thus, pathogenic forms of corneodesmosin which are associated with reduced proteolytic degradation may be identified by methods known in the art. These may correspond to the full length form (52-56 kDa) of the corneodesmosin protein in the superficial layers of the lesional stratum envelope. Pathogenic forms of the corneodesmosin may be identified by comparing proteolysis profiles from normal and lesional skin. For example, Western blot using protein extract from skin strips and probed with an antibody which recognises corneodesmosin may be used.

### ADHESION PROTEIN

Where the term "adhesion protein" is used in this document, this should be taken as reference to any protein, polypeptide or peptide which mediates adhesion between cells. Thus, an "adhesion protein" includes any protein which is involved in cell-cell adhesion.

Preferably, the adhesion protein mediates cell-cell interaction between epithelial cells, i.e., the adhesion protein is an epithelial cell adhesion protein.

Preferably, the adhesion protein mediates cell-cell interaction between epidermal cells. More preferably, the adhesion protein mediates cell-cell adhesion between corneocytes. Preferably, the adhesion protein is located in a comeodesmosome. Most preferably, the adhesion protein is a desmosomal protein or a corneodesmosomal protein.

The adhesion protein may suitably be selected from the group consisting of adhesion proteins shown in Tables D2.1 and 5.1. Preferably, the adhesion protein is selected from the group consisting of corneodesmosin (AF030130), desoplakin (XM_004463), plakoglobin (NM_002230); (NM_021991), desmoglein 1 (XM008810), desmocollin 1 (MX_008687), envoplakin (XM_008135;U72543), plectin 1 (NM000445), S100A2 (AI539439;M87068), keratin 6A (L42611), keratin 17 (Z19574), S100A8 (AI126134), S100A7 (AA586894), S100A9), GB:W72424), SPRR2A), GB:M21302), SPRRIB (M19888), SPRK (AI923984), HCR (BAA81890), SEEK1 (BAA88130), SPR1 (BAB63315), STG (BAA88132), involucrin (NM_005547), annexin Al/lipocortin (X05908), collagen, type VI, alpha 3 (COL6A3) (NM_004369), trichohyalin (NM_005547), and loricrin (XM_048902). GenBank accession numbers are provided in brackets.

Particularly preferred adhesion proteins comprise any of the desmosomal proteins corneodesmosin (also known as S; AF030130), desmoglein 1 (XM_008810), desmocollin 1 (MX_008687), desmoplakins I and II (XM_004463), plakoglobin (also known as PG; NM_002230) and plakophilin (also known as PP).

Highly preferred adhesion proteins include corneodesmosin, desmoglein I, desmoglein 3, plakoglobin, desmoplakin, desmocollin I, envoplakin, a proline-rich protein, preferably a small proline-rich protein (SPRR), SPRR2A, SPRR1 B, SPRK, SPRR2E, SPRR2F, SPRR2B, SPRR2D, SPRR2C, SPRR2G, SPRR1A, SPRR3, SPRR4, involucrin, or loricrin.

Mutations and polymorphisms in any of the above adhesion proteins which lead to or are associated with increased or decreased adhesion between epidermal cells may be detected by the methods described in detail in the Examples. Such changes may be detected as a means to identify or diagnose skin disease or susceptibility to such disease.

### Corneodesmosin

Mutations in adhesion proteins may be detected as a means to diagnose skin disease or susceptibility to such diseases. In a highly preferred embodiment, the adhesion protein is corneodesmosin.

The corneodesmosin gene is very polymorphic. To date there are nineteen variants described in the literature (Kasahara et al 1996, Jenisch et al, 1999). These polymorphisms are highly conserved rather than sporadic and we believe that they have been selected during vertebrate evolution. Because of the barrier function of the skin some forms of the corneodesmosin giving a strong resistance and/or dynamic function to the skin have been selected. A strong association has been shown between comeodesmosin variant at position +1243 and chronic plaque psoriasis (Tazi-Ahnini et al, 1999b). This association is even stronger in guttate psoriasis (Tazi-Ahnini et al, 1999b). The substitution at position +1243 gives amino acid change L394S. We believe that this substitution interferes with the processing of the corneodesmosin, thus contributing to the disruption of desquamation. We have found that nine of the comeodesmosin polymorphisms giving amino acid change (Ser143/Asp, Ser153/-, Leu180/Phe, Ser202/Phe, Ser401/Gly, Ser408/Ala, Gly409Nal, Ser410/Leu, Asp527/Asn) have an important function in keratinocyte maturation and desquamation process. The screening method for these polymorphisms is described by Jenisch et 1999 and Guerrin et al, 2000. We show that there is a strong relationship between proteolysis process of the comeodesmosin and the sensitivity of normal skin, and also with diseases including psoriasis, acne, eczema in which the barrier function is disturbed.

We show in the Examples that mutations in corneodesmosin are associated with skin diseases, particularly diseases of decreased adhesion.

In particular, we demonstrate in the Examples that a T nucleotide in position +1243 of the comeodesmosin sequence (AF030130) is associated with diseases of decreased skin adhesion. The presence of a T nucleotide at this position leads to a threonine (T) residue at position 394 of the encoded comeodesmosin polypeptide. Detection of either or both may be used to diagnosis disease.

We therefore disclose the diagnosis of a disease of decreased skin adhesion (a Group I disease) or susceptibility to such a disease in an individual, by detecting the presence of a T at position +1243 of a comeodesmosin nucleic acid in an individual. We also provide for the diagnosis of such a disease, or susceptibility to it, by detection of a threonine (T) residue at position 394 of a corneodesmosin polypeptide in an individual. Group I diseases may also be diagnosed by detection of a CD5 or CD6 corneodesmosin allele in an individual; the sequence of the CD5 and CD6 alleles is described in Jenisch et al. 1999.

Preferably, the Group I disease is eczema or dermatitis. More preferably, the Group I disease is atopic eczema or dermatitis hyperformis. Other examples of Group I diseases are set out in a separate section in this document.

Other mutations are disclosed in the Examples at +619 and +180 of corneodesmosin nucleic acid; these and the corresponding polypeptide changes may be detected to diagnose a Group I or Group II disease. Other diagnosis and treatment methods employing comeodesmosin are disclosed in the Examples.

### PROTEASES

Protease which may be used in the methods desribed here include the following:
*Stratum Corneum Chymotryptic Enzyme (SCCE)*
*Stratum Corneum Tryptic Enzyme (SCTE)*

Stratum corneum chymotryptic enzyme (SCCE) or kallikrein 7 (KLK7) as well as stratum corneum tryptic enzyme (SCTE) or kallikrein 5 (KLK5) are members of large serine protease family. Analysis of their expression profiles suggests their skin specificity. Both enzymes are expressed in high suprabasal keratinocytes and interfollicular epidermis and have maximum activity at the physiologic pH of stratum comeum (Ekholm et al, 2000; Eugelrud, 1992, Ekholm and Eugelrud, 1998).

SCCE and SCTE are transported to the stratum comeum extracellular space during cornification. SCCE is produced as an inactive precursor and there is a need for an activating enzyme to confer to SCCE its proteolytic activity. SCTE is thought to be the enzyme involved in SCCE activation. A cluster of kallikrein genes including SCCE and SCTE has been mapped in chromosome 19q13.3-13.4. This includes at least 6 structurally and evolutionary-related members (KLK1, KLK2, KLK3, KLK4, SCCE and SCTE). Each of these kallikrein genes may be used in the methods and compositions described here.

Of those SCCE and SCTE are mainly expressed in skin. They are also expressed in other tissues such as brain, kidney, mammary and salivary glands (Yousef et al, 2000; Yousef and Diamandis, 1999).

### PROTEASE INHIBITORS

We demonstrate that regulation of desquamation is not only controlled by proteases (for example., SCCE and SCTE) but also by their inhibitors. Protease inhibitors which may be used in the methods and compositions described here include the following:
*Secretory Leukoprotease Inhibitor (SLPI) and Elafin*

Several serine proteases are present in human epidermis including antileukoprotease (skin-derived antileukoprotease) and elafin. Antileukoprotease is a powerful inhibitor of SCCE. Elafin protease inhibitor 3 (PI3 or SKALP) is another serine proteases inhibitors produced by keratinocytes. It is over-expressed in the subcomeal layers of lesional psoriatic skin and in other skin disorders such as Behcet's syndrome, Sweet's syndrome, pyoderma gangrenosum and cutaneous allergic vasculitis (Tanaka et al. 2000). We have found that changes of SKALP expression affects SCCE and SCTE activities and hence disturbs the structure of superficial layers of the epidermis in skin disorders such as psoriasis and eczema.

The terms "Secretory Leukoprotease Inhibitor", "SLPI" and "antileukoprotease" as used in this document are intended to be synonymous with each other.

Elafin is also known as skin-derived antileukoprotease, SKALP and protease inhibitor 3 (PI3). Accordingly, these terms are intended to be synonymous to each other, as used in this document.

### POLYPEPTIDES

The methods and compositions described here provide generally for a number of polypeptides, together with fragments, homologues, variants and derivatives thereof. Suitably useful polypeptides include adhesion proteins, proteases or protease inhibitors, or fragments, homologues, varianst or derivatives thereof.

Thus, we disclose variants, homologues or derivatives of an amino acid sequence of adhesion proteins, protease or protease inhibitor, as well as variants, homologues or derivatives of a nucleotide sequence encoding such amino acid sequences. Each of these may be used for the diagnosis of a Group I diesease.

Preferably, the polypeptides, variants, homologues, fragments and derivatives disclosed here comprise one or more properties of the adhesion protein, protease or protease inhibitor, preferably one or more biological activities. Thus, the variants, etc preferably comprise one or more activities including but not limited to adhesion, protease and protease inhibitor activity.

### Homologues

The polypeptides disclosed include homologous sequences obtained from any source, for example related viral/bacterial proteins, cellular homologues and synthetic peptides, as well as variants or derivatives thereof Thus polypeptides also include those encoding homologues of an adhesion protein, protease or protease inhibitor from other species including animals such as mammals (e.g. mice, rats or rabbits), especially primates, more especially humans. More specifically, homologues include human homologues.

In the context of this document, a homologous sequence is taken to include an amino acid sequence which is at least 15, 20, 25, 30, 40, 50, 60, 70, 80 or 90% identical, preferably at least 95 or 98% identical at the amino acid level, preferably over at least 50 or 100, preferably 200, 300, 400 or 500 amino acids with the relevant sequence.

In particular, homology should typically be considered with respect to those regions of the sequence known to be essential for protein function rather than non-essential neighbouring sequences, for example, sequences essential for proteolysis and/or adhesion. This is especially important when considering homologous sequences from distantly related organisms.

Although homology can also be considered in terms of similarity (i.e. amino acid residues having similar chemical properties/functions), in the context of the present invention it is preferred to express homology in terms of sequence identity.

Homology comparisons can be conducted by eye, or more usually, with the aid 15 of readily available sequence comparison programs. These publicly and commercially available computer programs can calculate % identity between two or more sequences.

% identity may be calculated over contiguous sequences, i.e. one sequence is aligned with the other sequence and each amino acid in one sequence directly compared with the corresponding amino acid in the other sequence, one residue at a time. This is called an "ungapped" alignment. Typically, such ungapped alignments are performed only over a relatively short number of residues (for example less than 50 contiguous amino acids).

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment! is performed. Consequently, most sequence comparison methods are designed to produce optimal alignments that take into consideration possible insertions and deletions without penalising unduly the overall homology score. This is achieved by inserting "gaps" in the sequence alignment to try to maximise local identity or similarity.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A; Devereux et al., 1984, Nucleic Acids Research 12:387). Examples of other software than can perform sequence comparisons include, but are not limited to, the BLAST package (see Ausubel *et al.,* 1999 ibid — Chapter 18), FASTA (Altschul et al., 1990, J. Mol. Biol., 403-410) and the GENEWORKS suite of comparison tools. Both BLAST and FASTA are available for offline and online searching (see Ausubel *et al.,* 1999 *ibid,* pages 7-58 to 7-60). However it is preferred to use the GCG Bestfit program.

Although the final % homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Once the software has produced an optimal alignment, it is possible to calculate % homology, preferably % sequence identity. The software typically does this as part of the sequence comparison and generates a numerical result.

The terms "variant" or "derivative" in relation to the amino acid sequences of the present invention includes any substitution of, variation of, modification of, replacement of, deletion of or addition of one (or more) amino acids from or to the sequence providing the resultant amino acid sequence retains substantially the same activity as the unmodified sequence, preferably having at least the same activity as the polypeptides shown here.

Polypeptides having the amino acid sequence shown here, including in the Examples, or fragments or homologues thereof may be modified for use in the methods and compositions described here. Typically, modifications are made that maintain the biological activity of the sequence. Amino acid substitutions may be made, for example from 1, 2 or 3 to 10, 20 or 30 substitutions provided that the modified sequence retains the biological activity of the unmodified sequence. Alternatively, modifications may be made to deliberately inactivate one or more functional domains of the polypeptides described here. Functional domains of proteases include the protease domain and protease catalytic site. Amino acid substitutions may include the use of non-naturally occurring analogues, for example to increase blood plasma half-life of a therapeutically administered polypeptide.

Conservative substitutions may be made, for example according to the Table below Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

### Fragments

Polypeptides also include fragments of the full length sequence of any of the polypeptides described here (e.g., an adhesion protein, protease or protease inhibitor). Preferably fragments comprise at least one epitope. Methods of identifying epitopes are well known in the art. Fragments will typically comprise at least 6 amino acids, more preferably at least 10, 20, 30, 50 or 100 amino acids.

Included are fragments comprising, preferably consisting of, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69. 70, 71, 72, 73, 74, 75, 76, 77, 78, 79, 80 81, 82, 83, 84, 85, 86, 87, 88, 89, 90, 91, 92, 93, 94, 95 96, 97, 98. 99, 100, 105,110,115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 296, 300, 305, 310, 315, 320, 325, 330, 335, 340, 345, 350, 355, 360, 365, 370, 375, 380, 385, 390, 400, 405, 410, 415, 420, 425, 430, 435, 440. 445. 450 455, 460, 465, 470, 475, 480, 485, 490, 500, or more residues from a relevant nucleic acid sequence, e.g., a nucleic acid sequence encoding an adhesion protein, protease or protease inhibitor.

Adhesion proteins, proteases and protease inhibitors, and their fragments, homologues, variants and derivatives, may be made by recombinant means. However they may also be made by synthetic means using techniques well known to skilled persons such as solid phase synthesis. The proteins may also be produced as fusion proteins, for example to aid in extraction and purification. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence of interest to allow removal effusion protein sequences. Preferably the fusion protein will not hinder the function of the protein of interest sequence. Proteins may also be obtained by purification of cell extracts from animal cells.

The polypeptides, variants, homologues, fragments and derivatives disclosed here may be in a substantially isolated form. It will be understood that such polypeptides may be mixed with carriers or diluents which will not interfere with the intended purpose of the protein and still be regarded as substantially isolated. A variant, homologue, fragment or derivative may also be in a substantially purified form, in which case it will generally comprise the protein in a preparation in which more than 90%, e.g. 95%, 98% or 99% of the protein in the preparation is a protein.

The polypeptides, variants, homologues, fragments and derivatives disclosed here may be labelled with a revealing label. The revealing label may be any suitable label which allows the polypeptide, etc to be detected. Suitable labels include radioisotopes, e.g. ¹²⁵I enzymes, antibodies, polynucleotides and linkers such as biotin. Labelled polypeptides may be used in diagnostic procedures such as immunoassays to determine the amount of a polypeptide in a sample. Polypeptides or labelled polypeptides may also be used in serological or cell-mediated immune assays for the detection of immune reactivity to said polypeptides in animals and humans using standard protocols.

A polypeptide, variant, homologue, fragment or derivative disclosed here, optionally labelled, my also be fixed to a solid phase, for example the surface of an immunoassay well or dipstick. Such labelled and/or immobilised polypeptides may be packaged into kits in a suitable container along with suitable reagents, controls, instructions and the like. Such polypeptides and kits may be used in methods of detection of antibodies to the polypeptides or their allelic or species variants by immunoassay.

Immunoassay methods are well known in the art and will generally comprise: (a) providing a polypeptide comprising an epitope bindable by an antibody against said protein; (b) incubating a biological sample with said polypeptide under conditions which allow for the formation of an antibody-antigen complex; and (c) determining whether antibody-antigen complex comprising said polypeptide is formed.

The adhesion protein, protease or protease inhibitor polypeptides, variants, homologues, fragments and derivatives disclosed here may be used in in vitro or in vivo cell culture systems to study the role of their corresponding genes and homologues thereof in cell function, including their function in disease. For example, truncated or modified polypeptides may be introduced into a cell to disrupt the normal functions which occur in the cell. The polypeptides may be introduced into the cell by in situ expression of the polypeptide from a recombinant expression vector (see below). The expression vector optionally carries an inducible promoter to control the expression of the polypeptide.

The use of appropriate host cells, such as insect cells or mammalian cells, is expected to provide for such post-translational modifications (e.g. myristolation, glycosylation, truncation, lapidation and tyrosine, serine or threonine phosphorylation) as may be needed to confer optimal biological activity on recombinant expression products. Such cell culture systems in which the adhesion protein, protease and protease inhibitor polypeptides, variants, homologues, fragments and derivatives disclosed here are expressed may be used in assay systems to identify candidate susbtances which interfere with or enhance the functions of the polypeptides in the cell.

### FRAGMENTS

We also provide for nucleic acids and polypeptides or peptides which are fragments of the adhesion protein, protease or protease inhibitor nucleic acids and polypeptides disclosed here.

Preferably such nucleic acid and polypeptide fragments comprise, preferably consist of, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69. 70, 71, 72, 73, 74, 75, 76, 77, 78, 79, 80 81, 82, 83, 84, 85, 86, 87, 88, 89, 90, 91, 92, 93, 94, 95 96, 97, 98. 99, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 280, 285, 290, 296, 300, 305, 310, 315, 320, 325, 330, 335, 340, 345, 350, 355, 360, 365, 370, 375, 380, 385, 390, 400, 405, 410, 415, 420, 425, 430, 435, 440. 445. 450 455, 460, 465, 470, 475, 480, 485, 490, 500, or more residues from a relevant sequence (i.e., any of the sequences disclosed here). Preferably, such fragments comprise biological activity, preferably adhesion, protease or protease inhibitor activity.

A nucleic acid of use in the methods described here may comprise a viral or non-viral DNA or RNA vector, where non-viral vectors include, but are not limited to, plasmids, linear nucleic acid molecules, artificial chromosomes, condensed particles and episomal vectors. Expression of heterologous genes has been observed after injection of plasmid DNA into muscle (WolffJ. A. et al., 1990, Science, 247: 1465-1468; Carson D.A. et al., US Patent No. 5,580,859), thyroid (Sykes et al., 1994, Human Gene Ther., 5: 837-844), melanoma (Vile et al., 1993, Cancer Res., 53: 962-967), skin (Hengge et al., 1995, Nature Genet., 10: 161-166), liver (Hickman et al., 1994, Human Gene Therapy, 5: 1477-1483) and after exposure of airway epithelium (Meyer et al., 1995, Gene Therapy, 2: 450-460).

As used herein, the term "nucleic acid" is defined to encompass DNA and RNA or both synthetic and natural origin which DNA or RNA may contain modified or unmodified deoxy- or dideoxy- nucleotides or ribonucleotides or analogues thereof. The nucleic acid may exist as single- or double-stranded DNA or RNA, an RNA/DNA heteroduplex or an RNA/DNA copolymer, wherein the term "copolymer" refers to a single nucleic acid strand that comprises both ribonucleotides and deoxyribonucleotides.

The term "synthetic", as used herein, is defined as that which is produced by in vitro chemical or enzymatic synthesis.

### ANTIBODIES

We further provide an antibody, either polyclonal or monoclonal, which specifically binds to epitopes on the polypeptide/protein encoded by the corneodesmosin gene or mutant epitopes. In preparing the antibody, the protein (with and without mutations) encoded by the corneodesmosin gene and polymorphisms thereof is used as a source of the immunogen. Peptide amino acid sequences isolated from the amino acid sequence or mutant peptide sequences may also be used as an immunogen.

The antibodies may be either monoclonal or polyclonal. Conveniently, the antibodies may be prepared against a synthetic peptide based on the sequence, or prepared recombinantly by cloning techniques or the natural gene product and/or portions thereof may be isolated and used as the immunogen. Such proteins or peptides may be used to produce antibodies by standard antibody production technology well known to those skilled in the art as described generally in Harlow and Lane, Antibodies: A Laboratory Manual, Cold Spring Harbor Laboratory Press, Cold Spring Harbor, N.Y., 1988.

For producing polyclonal antibodies a host, such as a rabbit or goat, is immunized with the protein or peptide, generally with an adjuvant and, if necessary, coupled to a carrier; antibodies to the protein are collected from the sera.

For producing monoclonal antibodies, the technique involves hyperimmunization of an appropriate donor, generally a mouse, with the protein or peptide fragment and isolation of splenic antibody producing cells. These cells are fused to a cell having immortality, such as a myeloma cell, to provide a fused cell hybrid which has immortality and secretes the required antibody. The cells are then cultured, in bulk, and the monoclonal antibodies harvested from the culture media for use.

The antibody may be bound to a solid support substrate or conjugated with a detectable moiety or be both bound and conjugated as is well known in the art. (For a general discussion of conjugation of fluorescent or enzymatic moieties see Johnstone and Thorpe, Immunochemistry in Practice, Blackwell Scientific Publications, Oxford, 1982.) The binding of antibodies to a solid support substrate is also well known in the art. (see for a general discussion Harlow and Lane Antibodies: A Laboratory Manual, Cold Spring Harbor Laboratory Publications, New York, 1988) The detectable moieties may include, but are not limited to, fluorescent, metallic, enzymatic and radioactive markers such as biotin, gold, ferritin, alkaline phosphatase, J3galactosidase, peroxidase, urease, fluorescein, rhodamine, tritium, 14 C and iodination.

### DIAGNOSIS OF DISEASES

We provide for methods of diagnosis of diseases associated with abnormal epithelial cell-cell adhesion. Mutations in genes encoding adhesion proteins (for example, corneodesmosin) are shown to result in reduced adhesion between epithelial cells such as corneocytes. Some mutations in these genes may result in altered (typically reduced) expression of adhesion proteins, or in expression of adhesion proteins which have reduced adhesion activity or higher protease sensitivity. Other mutations in protease and protease inhibitor genes are also associated with disease, as shown in the Examples. Such mutations are typically associated with and may lead to Group 1 diseases, as the reduced cell-cell adhesion results in impaired barrier function of the epidermis.

Accordingly, a Group 1 disease may be diagnosed in a patient suffering or likely to suffer from the disease by determining the level of expression of an adhesion protein, in which a reduced level of expression of an adhesion protein is diagnostic of a Group 1 disease. Furthermore, we provide a method of diagnosis of a Group 1 disease by determining the adhesion activity of an adhesion protein involved in epithelial cell-cell adhesion, in which a reduced adhesion activity is diagnostic of a Group 1 disease. We further provide a method of diagnosis of a Group 1 disease by determining the protease sensitivity of an adhesion protein involved in epithelial cell-cell adhesion, in which an increased protease sensitivity is diagnostic of a Group 1 disease. We also disclose a method of diagnosis of a Group 1 disease, by determining the presence of a mutation which is associated with a reduced expression of an adhesion protein, or with expression of an adhesion protein with reduced adhesion activity and/or increased protease sensitivity.

Other mutations in genes encoding adhesion proteins such as corneodesmosin result in increased adhesion between epithelial cells such as comeocytes. Some mutations in these genes may result in enhanced expression of adhesion proteins, or in expression of adhesion proteins which have enhanced adhesion activity or reduced protease sensitivity.

Any method of determining protease sensitivity of a protein as known in the art (Egelrud, 1993) may be used in the diagnostic methods presented above. Similarly, detection of mutations by means of, for example, SSCP, RFLP, SNP, etc analysis are known in the art and are described in further detail below.

Detection of abnormal (i.e., increased or decreased) proteolytic breakdown of an adhesion protein may also be used to diagnose Group 1 diseases. Thus, an increased level of expression and/or activity of a protease involved in proteolysis of an adhesion protein is characteristic (and hence diagnostic) of a Group 1 disease. Accordingly, we provide a method of diagnosis of a Group 1 disease, by detecting the level of expression of a protease involved in proteolysis of an adhesion protein. We further provide a method of diagnosis of a Group 1 disease, by detecting the level of activity of a protease involved in proteolysis of an adhesion protein, in which an increased level of activity of the protease is diagnostic of a Group 1 disease. The level of expression and/or activity of a protease inhibitor which is capable of inhibiting the proteolytic activity of a protease involved in breakdown of an adhesion protein may also be detected as a means of diagnosis of a Group 1 disease. Accordingly, decreased level of expression and/or activity of a protease inhibitor is characteristic of a Group 1 disease. Therefore, a method of diagnosis of a Group 1 disease comprises detecting a decreased level of expression of a protease inhibitor in a patient. Similarly, a method of diagnosis of a Group 1 disease may also comprise detecting the level of activity of a protease inhibitor, in which an decreased level of activity of a protease inhibitor is diagnostic of a Group 1 disease.

It is clear that detection of expression and/or activity of proteases and/or protease inhibitors may also be undertaken at a genetic level, i.e., detecting mutations associated with increased or decreased protease/protease inhibitor activity.

Preferably, the adhesion protein which is detected or whose properties are determined in the above methods is corneodesmosin, and preferably, the gene is a gene encoding corneodesmosin. Preferably, the protease that is detected, etc is stratum corneum chymotryptic enzyme (SCCE) or stratum corneum tryptic enzyme (SCTE).

Preferably, the protease inhibitor that is detected, etc, is anti-leukoprotease (SLPI) or elafin protease inhibitor 3 (PI3 or SKALP).

Preferably, the protease inhibitor that is detected, etc, is or elafin. Where the terms "higher", "increased", and "enhanced" are used with reference to activity, protease sensitivity, expression, etc, these are understood to be relative to the corresponding properties of a normal, un-diseased epithelium from the same patient or another individual. Similarly, the terms "lower", "decreased" and "reduced" are to refer to a level relative to a normal, un-diseased epithelium.

Specific non-limiting examples of diagnostic methods suitable for the methods and compositions described here are set out below:

Proteolysis profiles may be conducted as follows. Polyclonal antibody capable of recognising the full and the mature forms of corneodesmosin are generated. Stratum corneum extract may be extracted from strips obtained from patients. Protein extracts are run on SDS-PAGE gel, transferred onto a membrane and hybridised with primary anti-corneodesmosin antibody and labelled secondary antibody. Molecular weight of processed corneodesmosin of patients are compared to the ones from controls.

Screening for N-/C-terminals may be conducted as follows. In the case of the presence of particular forms of desmosomal/corneodesmosomal proteins (e.g. corneodesmosin) in a patient group but not in controls or vice versa, N-/C-terminal specific antibodies antibody may be produced to detect the pathogenic forms of desmosomal/corneodesmosomal proteins (e.g. corneodesmosin). The identification of the pathogenic forms may be performed as follow. Different forms of corneodesmosin are purified from non denaturing hypotonic buffer extract (TEA buffer extract) of human epidermis by anion exchange and affinity chromatography (Simon et al, 1997).

The eluted fractions of the affinity column are pooled, lyophilized, separated by SDS-PAGE. The bands corresponding to the pathogenisis forms of corneodesmosin are excised and characterized by internal and NH2-terminal amino acid sequence analysis.

### DESMOSOMES AND CORNEODESMOSOMES

Desmosomes are symmetrical structures that form disc-shaped intercellular junctions between epithelial cells. In the epidermis the desmosomes mediate the adhesion between keratinocytes. In psoriasis, various ichtyoses and skin xerosis, the number of corneodesmosomes (desmosomes in upper layers of the epidermis) is increased in the stratum corneum. Immunoelectron microscopy has been used to show the define the interactions within corneodesmosomes between proteins of the extracellular core domain such as desmoglein and desmocollins and intracellular corneodesmosomal proteins including desmoplakins I and II, plakoglobin (PG) and plakophilins (PP) (Cowin and Burke, 1996). The importance of corneodesmosomal proteins in epidermal integrity is demonstrated by inherited disorders such as striate subtype of palmoplantar keratoderma caused by desmoplakin haploinsufficiency (Armstrong et al, 1999). Mutations in loricrin gene lead to keratoderma of Camisa. Corneodesmosin is a glycoprotein of corneodesmosomes. Three forms of the corneodesmosin with different weights 33-36 to 40-46 and 52-56 kDa have been isolated from the epidermis (Simon et al, 1997).

Mutations within the comeodesmosin/S gene and related genes within the MHC epidermal gene cluster (chromosome 6p21) result in a reduced cohesion between corneocytes.

### DIAGNOSIS

We disclose a method for diagnosing and detecting carriers of a defective an adhesion protein, protease or protease inhibitor gene responsible for causing Group 1 diseases, or associated with abnormal regulation of proteolysis of an adhesion protein such as corneodesmosin.

We further provide methods for detecting normal copies of the gene and its gene product. Identifying carriers either by their defective gene or by their missing or defective protein(s) encoded thereby, leads to earlier and more consistent diagnosis of gene carriers. Thus, since carriers of the disease are more likely to be prone to Group 1 diseases, better surveillance and treatment protocols may be initiated for them.

Briefly, the methods comprise the steps of obtaining a sample from a test subject, isolating the appropriate test material from the sample and assaying for the target nucleic acid sequence or gene product. The sample may be tissue or bodily fluids from which genetic material and/or proteins are isolated using methods standard in the art. For example, DNA may be isolated from blood.

More specifically, the method of carrier detection is carried out by first obtaining a sample of either cells or bodily fluid from a subject. Convenient methods for obtaining a cellular sample may include collection of either mouth wash fluids or hair roots. A cell sample could be amniotic or placental cells or tissue in the case of a prenatal diagnosis. A crude DNA could be made from the cells (or alternatively proteins isolated) by techniques well known in the art. This isolated target DNA is then used for PCR analysis (or alternatively, Western blot analysis for proteins) with appropriate primers derived from the gene sequence by techniques well known in the art. The PCR product would then be tested for the presence of appropriate sequence variations in order to assess genotypic disease status of the subject.

The specimen may be assayed for polypeptides/proteins by immunohistochemical and immunocytochemical staining (see generally Stites and Terr, Basic and Clinical Immunology, Appleton and Lange, 1994), ELISA, RIA, immunoblots, Western blotting, immunoprecipitation, functional assays and protein truncation test. In preferred embodiments, Western blotting, functional assays and protein truncation test (Hogervorst et al., 1995) are used. mRNA complementary to the target nucleic acid sequence may be assayed by in situ hybridization, Northern blotting and reverse transcriptase--polymerase chain reaction. Nucleic acid sequences may be identified by in situ hybridization, Southern blotting, single strand conformational polymorphism, PCR amplification and DNA-chip analysis using specific primers. (Kawasaki, 1990; Sambrook, 1992; Lichter et al, 1990; Orita et al, 1989; Fodor et al., 1993; Pease et al.,1994).

ELISA assays are well known to those skilled in the art. Both polyclonal and monoclonal antibodies may be used in the assays. Where appropriate other immunoassays, such as radioimmunoassays (RIA) may be used as are known to those in the art. Available immunoassays are extensively described in the patent and scientific literature. See, for example, U.S. Pat. Nos. 3,791,932; 3,839,153; 3,850,752; 3,850,578; 3,853,87; 3,867,517; 3,879,262; 3,901,654; 3,935,074; 3,984,533; 3,996,345; 4,034,074; 4,098,876; 4,897,219; 5,011,771 and 5,281,521 as well as Sambrook et al, 1992.

Current mutation data as described here indicate that Group 1 diseases are characterized by allelic heterogenicity. Thus, it is important for a successful mutation screen to be able to detect all possible nucleotide alterations in the corneodesmosin or other adhesion protein, protease or protease inhibitor gene, rather than being focused on a limited subset. Methods including direct sequencing of PCR amplified DNA or RNA or DNA chip hybridization (Fodor et al., 1993; Pease et al., 1994) may be applied along with other suitable methods known to those skilled in the art.

In order to use the methods for diagnostic applications, it is advantageous to include a mechanism for identifying the presence or absence of target polynucleotide sequence (or alternatively proteins). In many hybridization based diagnostic or experimental procedures, a label or tag is used to detect or visualize for the presence or absence of a particular polynucleotide sequence. Typically, oligomer probes are labelled with radioisotopes such as 32 P or 35 S (Sambrook,1992) which may be detected by methods well known in the art such as autoradiography. Oligomer probes may also be labelled by non-radioactive methods such as chemiluminescent materials which may be detected by autoradiography (Sambrook, 1992). Also, enzyme-substrate based labelling and detection methods may be used. Labelling may be accomplished by mechanisms well known in the art such as end labelling (Sambrook, 1992), chemical labelling, or by hybridization with another labelled oligonucleotide. These methods of labelling and detection are provided merely as examples and are not meant to provide a complete and exhaustive list of all the methods known in the art.

The introduction of a label for detection purposes may be accomplished by attaching the label to the probe prior to hybridization.

An alternative method includes the step of binding the target DNA to a solid support prior to the application of the probe. The solid support may be any material capable of binding the target DNA, such as beads or a membranous material such as nitrocellulose or nylon. After the target DNA is bound to the solid support, the probe oligomers is applied.

Functional assays may be used for detection of Group 1 carriers or affected individuals.

We also provide a kit for diagnosis and detection of a defective corneodesmosin gene. The kit includes a molecular probe complementary to genetic sequences of a defective corneodesmosin gene which causes a Group 1 disease and suitable labels for detecting hybridization of the molecular probe and the defective gene thereby indicating the presence of the defective gene. The molecular probe has a DNA sequence complementary to mutant sequences. Alternatively, the kit may contain reagents and antibodies for detection of mutant proteins.

A kit for detection and diagnosis of a defective adhesion protein, protease or protease inhibitor gene is also provided, comprising a molecular probe complementary to genetic sequences of a defective adhesion protein, protease or protease inhibitor gene which causes a Group 1 disease and suitable labels for detecting hybridization of the molecular probe and the defective gene thereby indicating the presence of the defective gene. The molecular probe has a DNA sequence complementary to mutant sequences. Alternatively, the kit may contain reagents and antibodies for detection of mutant proteins.

A few different methods are commonly used to analyze DNA for polymorphisms or mutations. The most definitive method is to sequence the DNA to determine the actual base sequence (Maxam and Gilbert, 1977; Sanger et al., 1977). Although such a method is the most definitive it is also the most expensive and time consuming method.

Restriction mapping analysis may also be used in analyzing DNA for polymorphisms. If one is looking for a known polymorphism at a site which will change the recognition site for a restriction enzyme it is possible simply to digest DNA with this restriction enzyme and analyze the fragments on a gel or with a Southern blot to determine the presence or absence of the polymorphism. This type of analysis is also useful for determining the presence or absence of gross insertions or deletions. Hybridization with allele specific oligonucleotides is yet another method for determining the presence of known polymorphisms.

Thus, specific DNA sequences in an individual, for example, a gene encoding corneodesmosin, may undergo many different changes, such as deletion of a sequence of DNA, insertion of a sequence that was duplicated, inversion of a sequence, or conversion of a single nucleotide to another. Changes in a specific DNA sequence may be traced by using restriction enzymes that recognize specific DNA sequences of 4-6 nucleotides.

Restriction enzymes cut (digest) the DNA at their specific recognized sequence, resulting in one million or so pieces. When a difference exists that changes a sequence recognized by a restriction enzyme to one not recognized, the piece of DNA produced by cutting the region are of a different size. The various possible fragment sizes from a given region therefore depend on the precise sequence of DNA in the region. Variation in the fragments produced is termed "restriction fragment length polymorphism" (RFLP). The different sized-fragments reflecting different variant DNA sequences may be visualized by separating the digested DNA according to its size on an agarose gel and visualizing the individual fragments by annealing to a radioactively labeled DNA "probe". Each individual may carry two different forms of the specific sequence. When the two homologues carry the same form of the polymorphism, one band is seen. More than two forms of a polymorphism may exist for a specific DNA marker in the population, but in one family just four forms are possible; two from each parent. Each child inherits one form of the polymorphism from each parent. Thus, the origin of each chromosome region may be traced (maternal or paternal origin).

Furthermore, RT-PCR may be carried out, followed by restriction endonuclease fingerprinting (REF). REF is a modification of the single-strand conformation polymorphism (SSCP) method, and enables efficient detection of sequence alterations in DNA fragments up to 2 kb in length (Liu and Sommer, 1995).

The use of mass spectrometry to determine the presence of polymorphisms within known genes is disclosed in United States Patent No. 5, 869, 242. Single strand conformational polymorphism (SSCP) analysis is a rapid and efficacious method for detecting polymorphisms (Dean et al., Cell 61:863, 1990; Glavac and Dean, Hum. Mutation 2:404, 1993; Poduslo et al., Am. J. Hum. Genet. 49:106, 1992). In SSCP, abnormal strand motility on a gel is associated with mutational events in the gene.

Genetic analysis of polymorphisms is disclosed in detail in United States Patent Nos. 5,552,28, 5,654,13, 5,670,33, 5,807,67, 5,858,66, 5,691,15, 5,922,57, 5,972,60, 6,136,53 and 5,955,26, among others.

Any of these methods described in the above references may be used in the diagnostic methods described here.

### EXAMPLES

### EXAMPLES A: ADHESION PROTEIN POLYMORPHISMS

### Example Al. Identification of Corneodesmosin (S) Gene Polymorphisms

Genomic DNA is extracted from whole blood according to standard protocols and stored at 100 ng/H.1. One reported exonic polymorphism giving a T to C transition at position +1243 is analysed (Ishihara et al, 1996, Tazi-Ahnini et al, 1999a, 1999b).

Primers S 15 (5'CATTGCATTCCAGCCAGTGG3') and S 16 (5'AACTGGAGCTGCTGCTGAAGGA3') are used to amplify the polymorphism locus (+1243). PCRs are prepared in bulk and aliquoted to 251.11 volumes comprising 50 mM KCL, 20 mM Tris-HCL, 1.5 mM MgCl2, 200 uM each dNTP, 1.2 4M each primer, 1 U of Taq polymerase (Gibco BRL, Paisley, UK) and 200 ng of genomic DNA. Thermocycling conditions are 2 mM at 95°C, 28 cycles of 1 min at 95°C, 1 min at 58°C and 15 s at 72°C. The amplifications are ended by 15 min at 72°C.

Restriction digests are performed in 20 p. reactions containing 10 p. of PCR products and 2.5 U ofHphI and appropriate manufacturer's buffer (New England Biolabs, Hitchin, UK) at 37°C overnight. Allelic discrimination is performed by electrophoresis using 2% agarose. Hphl digestion produces 123 + 89 by for allele 1, while it does not cut allele 2 (212 bp).

### Alleles

In the following Examples, "allele 1" refers to an allele in which the nucleotide residue at position +1243 is a C. The protein encoded by such an allele has a serine (S) residue at position 394 of the amino acid sequence employing the numbering of the polypeptide sequence having accession number L20815.

Similarly, "allele 2" refers to an allele in which the nucleotide residue at position +1243 is a T. The protein encoded by such an allele has a leucine residue at position 394 of a amino acid sequence, employing the numbering of the polypeptide sequence having accession number L20815.

Amino acid position numbering refers to the translation initiation codon (+1, ATG) of corneodesmosin sequence with GenBank accession numbers GB: L20815 or AF030130, as indicated. Thus, for example, a +1243 substitution C to T gives amino acid change from S to F at position 394 according to GB sequence L20815, while the same nucleotide substitution gives amino acid change from S to F at position 410 according to the GB sequence AF030130.

### Statistical Analysis

Disease and control populations are compared using 2x3 tables, and the odds ratio is calculated by comparing individuals homozygotic for allele 1 with that for carriage of the alternative allele (allele 2) in control and patients population. A x2 test for allele 2 is also carried out, weighted by number of putative disease susceptibility alleles in each genotype group.

### Example A2. Association of Corneodesmosin (S) Gene +1243 Polymorphisms with Atopic Eczema

In this Example, unless otherwise indicated, amino acid positions in the corneodesmosin sequence are provided with reference to the numbering in GenBank sequence L20815.

This Example demonstrates that corneodesmosin allele 2, in which the nucleotide residue of corneodesmosin at position +1243 is a T, leading to the presence of a leucine (T) residue at position 394 of the corneodesmosin polypeptide (L20815), is associated with atopic eczema.

The allelic distribution of +1243 polymorphism is assessed in both the atopic eczema and controls groups (n=154 and 550, respectively), as described above. Both controls and patients are in Hardy Weinberg equilibrium. Table A2.1 shows the observed (A) and the expected (B) values of each genotype in controls and atopic eczema patients groups.

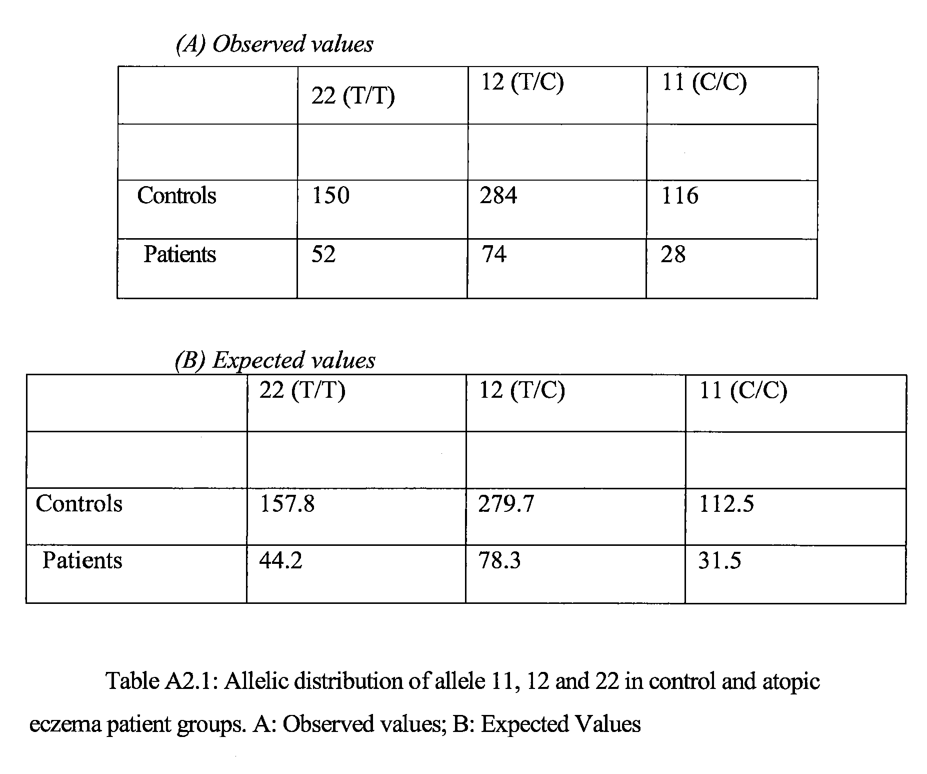

The overall difference between expected and observed values in the controls and patients is not statistically significant. However, there is an increase of the rare allele (allele 2, 52/44.2=1.2). Allele 1 (common allele) appears to be protective from eczema, and for this reason we group individuals with alleles 12 and 11 in one subgroup and 22 in the other (see Table A2.2 below).

**Table A2.2: Allelic distribution of allele 11 and 12/22 in control and atopic eczema patient groups**

| | | |
|---|---|---|
| | 22 (TT) | 11 / 12 (CC/CT) |
| Patients | 52 | 102 |

A x2 test for allele 2 against carriage of allele 1 is carried out. A significant association is found between allele 2 of the corneodesmosin gene (+1243) polymorphism and atopic eczema [OR =1.36 (0.93, 1.99)].

We therefore disclose the diagnosis of a Group I disease or susceptibility to a Group I disease (preferably eczema or susceptibility to eczema, preferably atopic eczema) in an individual, by detecting the presence of a T at position +1243 of a corneodesmosin nucleic acid, or the presence of a leucine (L) residue at position 394 of a corneodesmosin polypeptide (L20815), or both, of an individual.

### Example A3. Association of Corneodesmosin (S) Gene +1243 Polymorphisms with Dermatitis

In this Example, unless otherwise indicated, amino acid positions in the corneodesmosin sequence are provided with reference to the numbering in GenBank sequence L20815.

This Example demonstrates that corneodesmosin allele 2, in which the nucleotide residue of corneodesmosin at position +1243 is a T, leading to the presence of a leucine (L) residue at position 394 of the corneodesmosin polypeptide (L20815), is associated with dermatitis and might be the cause of the pathogenesis of dermatitis herpetiformis.

The allelic distribution of the +1243 polymorphism is assessed in both the dermatitis herpetiformis and controls groups (n=50 and 550, respectively), as described above. Both controls and patients are in Hardy Weinberg equilibrium.

**Table A3.1: Allelic distribution of allele 11, 12 and 22 in control and dermatitis herpetiformis groups**

| A) Observed values | | | |
|---|---|---|---|
| | 22 (T/T) | 12 (T/C) | 11 (C/C) |
| Controls | 150 | 284 | 116 |
| Patients | 26 | 23 | 1 |

| | | | |
|---|---|---|---|
| B) Expected values | | | |
| | 22 (T/T) | 12 (T/C) | 11 (C/C) |
| Controls | 161.3 | 281.4 | 107.2 |
| | | | |
| Patients | 14.6 | 25.5 | 9.7 |

There is an increase of the rare allele (allele 2, 26/14.6=1.78). Only one patient is homozygous for allele 1 (a common allele). This allele seems to be a protective since it is very frequent in control population.

**Table A3.2: Allelic distribution of allele 11 and 12/22 in control and dermatitis herpetiformis groups**

| | TT/CT (22/12) | CC (12/11) |
|---|---|---|
| Controls | 434 | 116 |
| Patients | 49 | 1 |

A x2 test for allele 1 against carriage of allele 2 is carried out. A significant association is found between allele 2 of S gene (+1243) polymorphism and dermatitis herpetiformis [OR =13.10 (1.79, 95.85); p<0.0001].

We therefore disclose the diagnosis of a Group I disease or susceptibility to a Group I disease (preferably dermatitis or susceptibility to dermatitis, preferably dermatitis herpetiformis) in an individual, by detecting the presence of a T at position +1243 of a corneodesmosin nucleic acid, or the presence of a leucine (L) residue at position 394 of a comeodesmosin polypeptide (L20815), or both, of an individual. This amino acid is at position 410 according to the GB sequence AF030130.

Allele 2 or T at position +1243 confers higher risk for dermatitis herpetiformis compared to atopic eczema because individual heterozygous at +1243 have also high risk of developing dermatitis.

### Example A4. Linkage Disequilibrium Analysis

In this Example, unless otherwise indicated, amino acid positions in the corneodesmosin sequence are provided with reference to the numbering in GenBank sequence L20815.

It has previously been shown that C at position +1243 is in linkage disequilibrium with T and G at position +619 and + 1240 respectively (Tazi-Ahnini et al, 1999a; Allen et al, 1999). The linkage disequilibrium is extended to other loci within the coding sequence of the corneodesmosin. Furthermore, using pedigree analysis with the transmission disequilibrium test (TDT), Jenisch and his colleagues identify 6 different alleles encoding 6 different amino acid sequences of the corneodesmosin gene (Jenisch et al, 1999).

### Group I Diseases

We show that within allele 5 (CDS) and allele 6 (CD6), T (leu) at position +1243 is in strong linkage disequilibrium with A (asp), AGT (ser), T (phe), A (ser), T (ser), T (leu), G (asp) and T (asn) of CD5 and A (asp), deletion (-), T (phe), A (ser), T (ser), T (leu), G (asp) and C (asp) of CD6. These variants are at position +442, + 468, +619, +1215, +1236, +1243, +1515 and +1593 respectively.

We find a strong association of these variants with atopic eczema in our collection. Therefore, detection of any of the changes listed above, and shown to be in linkage disequilibrium with T at position +1243 (i.e., allele 2), may be used in place of, or in addition to, detection of nucleotide T (+1243) or amino acid L (394 of L20815).

We therefore provide the diagnosis of a Group I disease or susceptibility to a Group I disease in an individual, preferably eczema or dermatitis, more preferably atopic eczema or dermatitis herpetiformis, or susceptibility to any of these diseases, by detecting any one or more of these changes.

Accordingly, we provide the diagnosis of a Group I disease, or susceptibility to a Group I disease, by detecting any one or more of the following residues at the relevant positions of a corneodesmosin nucleic acid, as shown in the Table A4.1 below.

**Table A4.1. Corneodesmosin Polymorphisms and Group I Diseases. (1): amino acid position numbering according to GenBank sequence L20815; (2): amino acid position numbering according to GenBank sequence AF030130.**

| Nucleotide Position | 442 | 468 | 619 | 1215 | 1236 | 1243 | 1515 | 1593 |
|---|---|---|---|---|---|---|---|---|
| Nucleic acid (s) | A | AGT | TA | | T | T | G | T |
| Residue Position (1) | 127 | 137 | 186 | 385 | 392 | 394 | 485 | 511 |
| Residue Position (2) | 143 | 153 | 202 | 401 | 408 | 410 | 501 | 527 |
| Residue | D | S/- | F | S | S | L | D | D/N |

### Summary

We have shown an association of a marker within the comeodesmosin gene giving an increased susceptibility to atopic eczema and dermatitis herpetiformis. It should be mentioned here that the comeodesmosin allele having C at +1243 (allele 1) is associated with psoriasis and acne vulgaris, suggesting that the two alleles give rise to different susceptibilities. Allele 2 (L394) is associated to barrier dysfunction (e.g. dermatitis, Crohn's disease) and allele 1 (5394) to impaired desquamation (e.g. psoriasis, acne).

We show association of polymorphisms at +1243 of the comeodesmosin gene with atopic eczema and dermatitis. The substitution at position +1243 gives amino acid change L394S. Without wanting to be bound by theory, we believe that this substitution interferes with the processing of comeodesmosin, thus contributing to enhanced desquamation.

We find that the comeodesmosin polymorphisms giving amino acid changes (Serl43/Asp, Ser153/-, Ser202/Phe, Ser401/Gly, Ser408/Ala,S410/L, Asp527/Asn; position according to GB sequence AF030130) or (Ser127/Asp, Serl37/-, Ser186/Phe, Ser385/Gly, Ser392/Ala, S394/L, Asp511/Asn; position according to GB sequence L20815) have an important function in the keratinocyte maturation and desquamation process. The screening method for these polymorphisms is described by Jenisch et al., 1999 and Guerrin et al, 2001. We therefore demonstrate that there is a strong relationship between the proteolytic processing of the comeodesmosin and the sensitivity of normal skin, and the integrity of diseased skin where there is a disruption in the barrier function including psoriasis, acne and dermatitis.

### Example A5. Corneodesmosin (S) Gene Polymorphism at Position 180

In this Example, unless otherwise indicated, amino acid positions in the comeodesmosin sequence are provided with reference to the numbering in GenBank sequence L20815.

### Identification of +180 Corneodesmosin Gene Polymorphism

The S gene variant at position 180 is identified by automatic sequencing of a cloned S allele (Guerrin et al, 2001). Nucleic acid change from C to T at position +180 gives an amino acid change from L at position 40 (L20815) to F. This amino acid is very conserved in mammalian species. Sequence alignments of human, mouse and pig S sequences show that L40 is conserved between these species as detailed below in Table A5.1.

**Table A5.1. Alignment of Corneodesmosin Sequences Amino acid number is provided with reference to the numbering of the sequence in GenBank accession number L20815. Nucleic acid substitution C to T at position 180 gives amino acid change L to F at position 56 of the comeodesmosin protein according to GB sequence AF030130.**

| | |
|---|---|
| Mouse | ITSPNDPCLI |
| Pig1 | IASPNDPCLL |
| Pig2 | IASP SDPCLL |
| Human | n-sPNDPCL₄₀T |

### Chymotrypsin Proteolysis

Peptides corresponding to fragments of comeodesmosin, and comprising amino acids at and around position 40 (L20815; corresponding to position 180 on the nucleotide sequence) are synthesised. The peptides are exposed to chymotrypsin in appropriate buffer, and the digestion products identified. Chymotrypsin is known to cleave the peptide bond C-terminal to a F, Y or W residue, but not C-terminal to an L residue.

Peptides P1 and P2 (size 38 amino acids, comprising L and F at position 40 of L20815 respectively) are exposed to chymotrypsin. Peptide P1 has the sequence PTRITSPNDPCL40TGKGDSSGFSGSSSSGSSISSAR, while peptide P2 has the sequence PTRITSPNDPCF40TGKGDSSGFSGSSSSGSSISSAR.

It is found that peptide P1 with L at position 40 of L20815 gives two products of proteolysis: PTRITSPNDPCL40TGKGDSSGF and SGSSSSGSSISSAR. However, peptide P2 with F at position 40 of L20815 gives three small peptides PTRITSPNDPCF, TGKGDSSGF and SGSSSSGSSISSAR. The above results are confirmed by using the ExPASy Molecular Biology Server at http://www.expasy.ch/. Peptides having the P1 and P2 sequences are predicted to be cleaved differently by chymotrypsin.

This demonstrates that the L to F amino acid change creates a new chymotrypsin site within corneodesmosin. This has an important effect on the corneodesmosin maturation during keratinocyte differentiation and desquamation. We therefore disclose an association of a L to F amino acid polymorphism in corneodesmosin and a disease phenotype.

We therefore disclose a method for the diagnosis of a Group I disease or susceptibility to a Group I disease (preferably dermatitis or susceptibility to dermatitis, preferably dermatitis herpetiformis, preferably eczema, preferably atopic eczema) in an individual, by detecting the presence of a protease cleavage site in a corneodesmosin polypeptide of an individual. Detection of the protease site may be done on the polypeptide sequence, or detecting a nucleic acid change which encodes a protease site. Preferably, the protease cleavage site is a cleavage site of SCCE or SCTE.

### Group I Diseases

We find in particular that the amino acid substitution from L to F at position 40 is associated with Group I disease (e.g., eczema and/or dermatitis). This substitution is caused by a change from C to T at position 180 of the corneodesmosin nucleic acid.

We therefore provide for the diagnosis of a Group I disease or susceptibility to a Group I disease (preferably dermatitis or susceptibility to dermatitis, preferably dermatitis herpetiformis, preferably eczema, preferably atopic eczema) in an individual, by detecting the presence of an F at position 40 of a corneodesmosin polypeptide of an individual.

We further provide for the diagnosis of a Group I disease or susceptibility to a Group I disease (preferably dermatitis or susceptibility to dermatitis, preferably dermatitis herpetiformis, preferably eczema, preferably atopic eczema) in an individual, by detecting the presence of a T at position 180 of a comeodesmosin nucleic acid of an individual.

### Example A6. Identification of +619 Corneodesmosin Gene Polymorphism

We demonstrate that the nucleic acid substitution C to T at position +619 gives an amino acid change from S to F at position 186 according to the numbering in L20815 and position 202 according to the numbering in AF030130. This creates a new chymotryptic site within the corneodesmosin protein. We find that the serine at position 186 is very conserved in mammalian species.

We further demonstrate that F at position 186 is associated with defective skin barrier such as in eczema and dermatitis. F at position 186 is found in CD5 and CD6 of the corneodesmosin polypeptide.

### Group I Diseases

We therefore provide for the diagnosis of a Group I disease or susceptibility to a Group I disease (preferably dermatitis or susceptibility to dermatitis, preferably dermatitis herpetiformis, preferably eczema, preferably atopic eczema) in an individual, by detecting the presence of an F at position 186 of a comeodesmosin polypeptide of an individual.

We further provide for the diagnosis of a Group I disease or susceptibility to a Group I disease (preferably dermatitis or susceptibility to dermatitis, preferably dermatitis herpetiformis, preferably eczema, preferably atopic eczema) in an individual, by detecting the presence of a T at position 619 of a comeodesmosin nucleic acid of an individual.

### EXAMPLES C: PROTEOLYSIS PROFILES/ PROTEASE MEDIATED MATURATION OF DESMOSOMAL AND CORNEODESMOSOMAL PROTEINS

### Example Cl. Production of Recombinant SCTE

Stratum Corneum Tryptic Enzyme (SCTE) is cloned and expressed in an active form such that it has full enzymatic activity.

### Expression Vectors Used

Expression is achieved using different vector systems in order to achieve protein expression at a high level, and enable purification. Three vector systems are used

*Insect Cell System:* No His-Tag at terminus; Invitrogen vector - pMTNS-HisA, B or C are used, depending on the reading frame. Expression in S2 insect (Drosophila) cells; purification as described in Hansson et al., 1994.

*Retroviral System:* No His-Tag; Clontech retroviral vectors (pLNCX2) Protein is expressed in murine mammary cells, and purified as described in Hansson et al., 1994.

*pcDNA3.l System:* His-Tag at the C-terminal; Invitrogen vectors (pcDNA3. 1, ABC with three ORFs) used. Expression of protein is achieved in COS-7 cells (Monkey African Green Kidney Cells).Reverse Transcription-Polymerase Chain Reaction (RT-PCR)

PCR Primers, covering the open reading frame, are designed manually i.e. by sight using SCTE sequence (GB: AF168768). All primers are from Invitrogen
*Forward* (5' TO 3'): GGA AAT CAG GTG CAG CG
*Reverse* (5' TO 3'): GAT GAC TCA GGA GTT GGC

Human epidermis total RNA ISreverse transcribed to cDNA with the Applied Biosystems Gold RNA PCR Kit. The RT Cycle comprises hybridisation for 10 mins at 25°C, followed by Reverse Transcription for 12mins at 42 °C. PCR is performed using Applied Biosystems Gold Taq Polymerase with 2.0mM Mg2+, 40 cycles. Each PCR Cycle consists of Taq Activation 95°C for 10mins, Denaturation: 94 for 30 sec, Annealing: 58.8 °C for 1 min, Extension: 72 °C for 20 seconds and Annealing/Extension: 72 °C for 7mins.

PCR amplification is verified on a 1.5% agarose gel. PCR products are purified from the other PCR mixture components using the Stratagene Strataprep PCR Purification Kit. The purified SCTE PCR product is used as the template for restriction enzyme PCRRestriction Enzyme Site Flanked PCR.

The insect cell vector pMT/VS-His (Invitrogen, Cat #K4120-20) is chosen for cloning This vector is available in 3 reading frames, A, B and C. Each reading frame facilitates cloning with expression of the C-terminal peptide. The correct reading frame is chosen with respect to the start codon of the SCTE DNA insert.

Restriction enzyme (RE) sites around the multiple cloning site of the vector, which are in frame with the SCTE start codon, are chosen. The SCTE DNA sequence is screened for the presence of restriction enzyme sites using the Webcutter program found on the NIH web site (www.nih.go.jp/—jun/research/anal.html). Those restriction enzyme sites not found in the SCTE sequence, but found on the vector are potentially available for use.

The SCTE start codon is found to be in frame with the pMT/VS-HisC vector. Kpnl is chosen as the first restriction enzyme, at the start codon. Notl is chosen as the second primer, after the stop codon. Restriction enzyme sites are incorporated into the forward and reverse SCTE PCR primers in order to generate a SCTE PCR product flanked by Kpnl and Not 1. These sites enable the sequence to be inserted into the vector:
*SCTE Forward Restriction Enzyme Primer*
(*S' to 3*) ATTAGTA-GGGTAG-ATGGCTACAGCAAGACCC

Random Sequence-Kpnl Restriction Enzyme Sequence-SCTE Start codon sequence
*SCTE Forward RE Primer (5' to 3* )
*ATTAGTA GGTACC ATGGCTACAGCAAGACCC*
*- Random SequenceKpnl RE Seq SCTE Start codon sequence*
*SCTE Reverse RE Primer (3 ' to* 5)

1) TCCAGGCCAACTC CTGA GCGGCCGC ATATTA
2) SCTE Stop codon sequence Notl RE Seq Random Sequence
   *SCTE Reverse RE Primer (5' to 3*) *(compliment)*
   TAATATGCGGC CGCTCAGGAG TTGGCCTGGA

SCTE PCR product from standard primer PCR is used as the template for Restriction Enzyme-primer PCR. PCR conditions are 2.5mM Mg2+ at any annealing temperature between 57 °C and 67 °C. The SCTE-Restriction Enzyme PCR product is digested with the corresponding restriction enzymes (Kpnl and Notl) according to the manufacturers recommended protocol (Promega). The digested SCTE-Restriction.

Enzyme PCR product is purified from the restriction enzyme mix by agarose gel electrophoresis and the DNA purified from the gel using the Qiagen Qiaexll Agarose Gel Extraction Kit. The SCTE insert is now ready for ligation to the prepared vector.

*PMTIV 5-HisC Vector Propagation and Restriction Enzyme*

### Digestion

The pMT/VS-HisC vector is propagated in Invitrogen TOP 10 chemically modified E.Coli Cells (Invitrogen, Cat #C4040-10). The protocol is described in the TOP 10 information booklet: Add 1p1 of the vector to a vial of TOP 10 cells (which have been thawed on ice). Gently mix. Incubate for 5 to 30mins on ice. Heat-shock cells for 30 seconds at 42 °C, without shaking. Immediately transfer back to ice. Add 250111 of room temperature SOC buffer. Cap the tube tightly and shake to tube horizontally at 37 °C for 1hour. Evenly spread 500 of transformant mix onto a fresh agar plate containing ampicillin. Incubate overnight at 37 °C. Single colonies are picked up and used to inoculate ampicillin-LB, and the culture incubated overnight at 37 °C. Glycerol stocks are made of the cultures. The propagated pMT/VS-HisC vector is purified from the bacteria using the Qiagen DNA Mini-Prep Kit, according to the manufacturers recommended protocol.

Purified vector is digested with Kpnl and Notl, as for the SCTE-Restriction Enzyme PCR product. Digested vector is also purified from the restriction enzyme mix by agarose gel electrophoresis and the DNA purified from the gel using the Qiagen QiaExII Agarose Gel Extraction Kit. The vector is now ready for ligation to the prepared SCTE insert.

### pMT/V5-HisC and SCTE Vector Ligation

The SCTE is ligated to the pMTN5-HisC vector using the enzyme T4-DNA Ligase (Gibco, Cat #15224-017). Ligation performed at a ratio of 5:1, DNA insert:vector according to the following protocol: DNA Ligase (1 U/1.11) 1[11, 5 x Ligation Buffer 4p1, SCTE Insert (X tig/p,1), pMT vector ((X .tg/i.i.1), Water (up to 201.1.1). The ligation reaction is incubated at 16 °C overnight (floating on icey water at 16 °C). The ligation reaction is assessed by 0.7% agarose gel (compared to unligated control samples). Competent E-Coli cells are transformed with ligated SCTE/Vector construct for propagation.

Propagation of SCTE/Vector Construct in E-Coli CellsTOP10 cells are transformed with the construct according to the manufacturers protocol, described above.The transformed cells are streaked onto ampicillin-agar plates and incubated overnight at 37°C. Single colonies are used to inoculate ampicillin-LB and the culture incubated overnight at 37 °C. Glycerol stocks have been made of the cultures. The construct is purified using the Qiagen DNA Mini-Prep Kit.

### Lipid Transfection of S2 Insect Cells

S2 Insect cells (drosophila, from Invitrogen, Cat #R690-07) are cultured as recommended by the manufacturer — in Drosophila Expression Medium (DES, Cat #Q500-01) supplemented with foetal calf serum and penicillin-streptomycin. Transfection is performed using Cellfectin (Invitrogen, Cat #10362-O10), a liposome formulation, again according to the manufacturers protocol.

### Induction of SCTE Protein Expression/Formation of Stable Cell Lines

The pMTN5-HisC vector has a metal-inducible (inducible with copper sulphate) promoter, metallothionein (MT), which enables high level expression of the target gene in S2 cells. Protein expression in the cells is induced with copper sulphate, added to a final concentration of 5001.1,M. Cells are incubated for 1-4 days. Following induction, cells are harvested and stored until further analysis. Polyacrylamide gel electrophoresis (PAGE) analysis is used to determine whether recombinant protein expression has occurred by comparing the total protein of induced cells and non-induced cells.

### Formation of Stable Cell Lines

After demonstrating that the SCTE protein is expressed in S2 cells, expression is scaled up by creating stable cells lines. Cells produce blasticidin, which is a potent translational inhibitor in prokaryotic and eukaryotic systems. Resistance to blasticidin is conferred after co-transfection of the SCTE/ pMT/VS-HisC construct and pCoBlast selection vector (Invitrogen, Cat #1K5150-01) into S2 cells. The protocol is found in the Drosophila expression system (DES) manual, which accompanies all DES reagents. Blasticidin (25ng/ml) is used to select stable transfectants.

### Scale-up of Protein Expression

Expression is scaled up for protein purification by using larger volumes/flasks. The Invitrogen manual enclosed with the S2 cells provides details of a protocol for doing this. Purification is performed by FPLC. Different fractions are collected and analysed for the presence of the recombinant protein.

### Example C2. Production of Recombinant SCCE

Stratum Corneum Chymotryptic Enzyme (SCCE) is cloned and expressed in an active form such that it has full enzymatic activity.

### Reverse Transcription-Polymerase Chain Reaction (RT-PCR)

PCR Primers, covering the open reading frame, are designed manually i.e. by sight using SCCE sequence (GB: NM005046). All primers are from Invitrogen.
*Forward* (5' TO 3'): CGG GCT CCA TGG CAA GAT C
*Reverse* (5' TO 3'): GCG TCC TCA CTC CTG TGC

Human epidermis total RNA reverse transcribed to cDNA with the Applied Biosystems Gold RNA PCR Kit. RT Cycle is as previously described for SCTE, using 2.0mM Mg2+, 40 cycles, PCR Cycle is as described for SCTE, except that annealing is done at 60oC for 1 min.

### Restriction Enzyme Site Flankd PCR

The correct reading frame and Restriction Enzyme sites around the multiple cloning site is chosen with respect to the start codon of the SCCE DNA insert. The SCCE DNA sequence is screened for the presence of restriction enzyme sites using the Webcutter program, and restriction enzyme sites chosen as described previously. The SCCE start codon is found to be in frame with the **pMT/VS-HisA vector**. EcoR1 is chosen as the first restriction enzyme, at the start codon. Xho1 is chosen as the second primer, after the stop codon.

Restriction enzyme sites are incorporated into the **forward and reverse SCCE PCR primers** in order to generate a SCCE PCR product flanked by EcoRl and Xhol. These sites enable the sequence to be inserted into the vector:-SCCE Forward Restriction Enzyme Primer (5' to 3')
GCC AGC -GAA TTC -ATGGCA AGA TCC CTT CTC

Random Sequence-EcoRl Restriction Enzyme Sequence-SCCE Start codon sequence
*SCCE Reverse Restriction Enzyme Primer (3' to 5* )
ATGAAAAAGCATCGCTAA-CTCGAG-AGCACT

SCCE Stop codon sequence-Xhol Restriction Enzyme Sequence-Random Sequence
*SCCE Reverse Restriction Enzyme Primer (5' to 3*) *(compliment)*
AGTGCTCTCG AGTTAGCGAT GCTTTTTCAT

SCCE PCR product from standard primer PCR is used as the template for Restriction Enzyme-primer PCR. PCR conditions are 2.0mM Mg2+ at an annealing temperature of 62 °C. The SCCE-Restriction Enzyme PCR product is digested with the corresponding Restriction Enzyme's (EcoRl and Xhol) — according to the manufacturers recommended protocol (Promega). The digested SCCE-Restriction Enzyme PCR product is purified from the restriction enzyme mix by agarose gel electrophoresis and the DNA purified from the gel using the Qiagen QiaexII Agarose Gel Extraction Kit. The SCCE insert is now ready for ligation to the prepared pMT/V5-HisA vector.

Expression is scaled up for protein purification by using larger volumes/flasks. The Invitrogen manual enclosed with the S2 cells provides details. Purification is performed by FPLC. Different fractions are collected and analysed for the presence of the recombinant protein. Further details for expression and purification of SCCE are disclosed in Hansson et al., 1994 (J. Biol. Chem. 269 (30), 19420-19426), which describes the purification of the protease enzyme SCCE.

### Example C3. Cleavage of Adhesion Proteins with Recombinant SCCE and SCTE

Recombinant ProSCCE is produced and purified as described above. Recombinant Pro SCCE is activated with agarose-bound trypsin, as described in 15 Brasttsand & Egelrud, 1999.

Proteins are extracted from human epidermis in the presence of a detergent (TENP-40 buffer extract) and incubated with active SCCE for 37C for 4 h. The reactions are stopped by boiling for 2 mins after Laemmli's sample buffer is added. The proteins are immunoblotted with antibodies against corneodesmosomal proteins.

### Cleavage of Adhesion Proteins by SCCE

We demonstrate with this Example that recombinant SCCE is able to cleave adhesion proteins, in particular, Corneodesmosin, Plakoglobin Desmoglein, Desmoplakin, Envoplakin and Desmocollin Therefore, these adhesion proteins are substrates of this protease.

SCCE and Corneodesmosin: Corneodesmosin is found to be proteolysed by recombinant SCCE. The native form of corneodesmosin has a molecular weight of between 50 and 56 kDa. After 2h incubation the major forms of Corneodesmosin are 36 and 46-43 kDa.

SCCE and Plakoglobin: Plakoglobin is found to be proteolysed by recombinant SCCE. The native form of plakoglobin has a molecular weight of between 85 kDa and 75 kDa. After 2-4 h incubation the major form of Plakoglobin is 70 kDa

SCCE and Desmoglein: Desmoglein is found to be proteolysed by SCCE. After 2-4h incubation the major forms of Desmoglein are 95 and 80 kDa which are the 10 proteolytic products of the 160 kDa form.

SCCE and Desmoplakin: Desmoplakin is found to be proteolysed by SCCE. The native form of Desmoplakin has a molecular weight of between 190-250 kDa. After 2-4 hour incubation the major forms of desmoplakin are 120-180 kDa and 75-80 kDa.

SCCE and Envoplakin: Envoplakin is found to be proteolysed by SCCE. The native form of Envoplakin has a molecular weight of between 124-209 kDa. After 2-4 hour incubation the major forms of envoplakin are 100-120 kDa, 60-80 kDa and 50-55 kDa.

SCCE and Desmocollin: Desmocollin is found to be proteolysed by SCCE. The 20 native form of Desmocollin has a molecular weight of between 70-80 kDa. After 2-4 hour incubation the major forms of desmocollin are 60-70 kDa and 50-60 kDa.

### Cleavage of Adhesion Proteins by SCTE

We demonstrate with this Example that recombinant SCTE is able to cleave adhesion proteins, in particular, Comeodesmosin, Plakoglobin Desmoglein, Desmoplakin, Envoplakin and Desmocollin. Therefore, these adhesion proteins are substrates of this protease.

SCTE and Comeodesmosin: Cornodesmosin is found to be proteolysed by recombinant SCTE. After 2h incubation the major forms of Comeodesmosin are 36 5 and 46-43 kDa.

SCTE and Plakoglobin: Plakoglobin is found to be proteolysed by recombinant SCTE. After 2-4 h incubation the major form of Plakoglobin is 70 kDa.

SCTE and Desmoglein: Desmoglein is found to be proteolysed by SCTE. After 2-4h incubation the major forms of Desmoglein are 95 and 80 kDa which are the 10 proteolytic products of the 160 kDa form.

SCTE and Desmoplakin: Desmoplakin is found to be proteolysed by SCTE.

After 2-4 hour incubation the major forms of desmoplakin are 120-180 kDa and 75-80 kDa.

SCTE and Envoplakin: Envoplakin is found to be proteolysed by SCTE. After 15 2-4 hour incubation the major forms of envoplakin are 100-120 kDa, 60-80 kDa and 50-55 kDa.

SCTE and Desmocollin: Desmocollin is found to be proteolysed by SCTE. After 2-4 hour incubation the major forms of desmocollin are 60-70 kDa and 50-60 kDa.

### Example C4. Proteolysis Profiles Materials and Methods

### Polyclonal Antibody Production

Antibodies to corneodesmosin, SCCE, envoplakin, desmoplakin, desmocollin 1 and SLPI are produced in rabbits by injection of synthetic peptides conjugated with keyhole limpet hemocyanin (KLH).

Peptides are coupled to a keyhole limpet hemocyanin (KLH) using standard procedures. The peptides are designed comprising the following amino acid sequences, derived from the relevant GenBank sequences as shown in Table C4.1below:

**Table C4.1. Bold C residues are synthesised for coupling and do not exist in the native sequence.**

| Protein | Peptide Sequence | Name | GenBank Sequence |
|---|---|---|---|
| Comeodesmosin | FTKENPVKGSPGVC | SB2 | GB: AF030130 |
| SCCE | INDTMKKHR | SB1 | GB: XM 009002 |
| SCCE | RRAQRIKASKS | SB4 | GB: XM 009002 |
| Envoplakin | SASPTVPRSLR | SB3 | GB: XM 008135 |
| Desmoplakin | SGKRDKSEEVQC | 642 | GB: XM 004463 |
| Desmocollin 1 | MENSLGPFPQC | 641 | GB: XM 004463 |
| SLPI | CGKSCVSPVKA | 644 | GB: X04502 |

Rabbits are injected with peptide coupled to KLH Rabbits at day 1. A brief protocol follows. Day 1: mix approximately 1501.11 (containing 3001.µg of conjugate) with an equal volume of Freunds Complete Adjuvant by passing several times through a 23G needle until an emulsion (which does not separate on standing) is formed. Inject subcutaneously into the rabbit using a 25G needle. Day 22, repeat the above but use Freunds Incomplete Adjuvant. Day 43, repeat the above exactly. Day 53, take first test bleed from ear vein. Boost and re-bleed as necessary.

### Monoclonal Antibodies

Monoclonal antibodies against adhesion protein antigens are obtained commercially from Biotechnic, Germany. These comprise anti-plakoglobin antibody directed against PG5.1 (Plakoglobin (NM-021991)) and anti-desmoglein 1 (XM-008810) antibody directed against Dsgl and Dsg2.

### Protein Extraction from the Epidermis

Proteins are extracted from biopsies in the form of "dog ears", which are triangular pieces of skin removed to produce a neat linear scar. This is not skin removed from the mastectomy specimen sent to the pathologists for diagnostics. All samples are from female patients attending clinics at the University of Sheffield undergoing mastectomy for breast carcinoma. Informed consent is obtained from the patients.

The epidermis is separated from the dermis by incubating a breast biopsy from breast surgery with trypsin solution A (Life Technology, France) for 18 h at 4 C. The epidermis is divided on two parts. One part is used for protein extraction and the other for RNA extraction (see below). The peeled epidermis is heated 5 mins in phosphate-buffuered saline at 56C (Simon et al, 1997). The epidermis is homogenized on ice on in equal volume of the following buffers (three times in each buffer): 40 mM Tris-HC1, pH 7.5, 10 mM EDTA, 0.25 mM phenylmethylsulfonyl fluoride, and 21 Ag/ml each of aprotinin, pepstatin A, and leupeptin (TE buffer), TE buffer containing 0.5% Nonidet P-40 (TE-Nonidet P-40 buffer).

The pellet is then divided into three parts that are extracted in one-third of the original volume of TE buffer containing various concentrations of urea (4, 6, and 8 M) (TEU buffers). After each extraction, the homogenates are centrifuge for 15 mins at 15,000 x g, and supernatants are kept at —30C until used. Finally, the pellet corresponding to the last extraction in 8 M urea is homogenized in 35 mM Tris-HC1, pH 6.8, 8M urea, 50 mM dithiothreitol, 5% glycerol, 0.25 mM phenylmethylsulfonyl fluoride, and 2 µg/m1 each of aprotinin, pepstatin A, and leupeptin (TUDTT buffer), incubated at 95C for 30 mins, and centrifuge as described above (this method is originally described by Simon et al, 1997). Protein concentrations are measured using the Coomassie Plus protein assay (Pierre Chemical Co, Rockford, IL).

### Protein Extraction from Stratum Corneum

Adhesion proteins are extracted from the stratum corneum according to a method described by Guerrin et al, 1998. Briefly, a tape strip is applied to a biopsy from normal skin or lesional and non-lesional of a patient with psoriasis. The tape strips are incubated in acetone and the tissue is recovered by centrifugation (500 x g, 1min), washed in acetone, and air-dried. The powder is boiled for 10 mins in 62.5 mM Tris-HC1, pH 6.8, containing 2% SDS and 50 mM DTT, and the solution is centrifuge (10,000 x g, 10 mins).

Proteins are extracted from the stratum corneum of normal and psoriatic patient groups, as described above, and analysed by Western blots using specific antibodies against corneodesmosomal proteins. We show that the profile of epidermally extracted proteins differs between normal and diseased (psoriatic) individuals.

### Western blot

Proteins from the epidermal and stratum corneum biopsies (—lidg) are separated by 10% SDS-polyacrylamide gel electrophoresis (SD S-PAGE). After electrophoresis the proteins are electrotransferred to nitrocellulose membranes. Membranes are blocked overnight at 4°C in Blotto (3% milk powder, 2% BSA, 0.1% Tween 20 in TBS). The membranes are probed with the primary antibodies described below for 3 hours at room temperature with agitation. Mouse monoclonal antibodies directed against plakoglobin and desmoglein are obtained from Progen (Heidelberg, Germany) and used at a 5p.g/ml concentration, diluted in Blotto. Rabbit polyclonal antibodies directed against peptides designed from protein sequences of desmocollin, desmoplakin, SLPI, SCCE, S protein and envoplakin (Antibody Resource Centre, Sheffield University, UK) are used at a 1:250 dilution. Membranes are washed for 3 x 5mins in Blotto and incubated in the presence of the secondary antibody, either anti- mouse or anti-rabbit IgG HRP obtained from Santa Cruz Biotechnology, Inc. (California, USA) at a 1:1000 dilution for 1 hour at room temperature, with agitation. The membranes are washed in TBS-Tween 20 (0.1 %) for 3 x 5 mins and proteins detected using the ECL+Plus Tm western blotting detection system (Amersham Pharmacia Biotech, Little Chalfont, UK).

### Example C5. Eczema Proteolysis Profiles of Proteins Extracted from the Stratum Corneum

Proteins are extracted from the stratum corneum of normal and eczematous patient groups, as described above, and analysed by Western blots using specific antibodies against corneodesmosomal proteins. We show that the profile of epidermally extracted proteins differs between normal and diseased (eczema) individuals.

### Corneodesmosome Proteolysis Profile

The increase in proteolysis of desmo/corneodesmosomal proteins in eczema skin is reflected in a significant increase in the quantity of immature forms of comeodesmosome proteins in eczematous skin compared to normal. Increased proteolysis causes the cells to stick together in the surface of the epidermis preventing cell shedding.

For example, the 36 and 46-43 kDa forms of corneodesmosin are the major forms in the stratum comeum in normal skin and the 52-56 kDa forms are very rare in the stratum corneum There is an increase of corneodesmosin proteolysis in eczematous skin, so that the 36 and 46-43 kDa forms of corneodesmosin are dominant forms in the stratum comeum of eczema patients. This shows that there is an increase in proteolysis in eczematous skin.

We therefore disclose the diagnosis of a Group I disease or susceptibility to a Group I disease (preferably eczema or susceptibility to eczema) in an individual, by detecting any of the changes described above, preferably the presence of or a modulated level, preferably a higher level of one or more 36, 46-43 kDa corneodesmosin polypeptides in an individual. We also disclose the diagnosis of a Group I disease or susceptibility to such a disease (preferably eczema or susceptibility to eczema) in an individual, by detecting the absence of or a modulated level, preferably a lower level of 52-56 kDa comeodesmosin polypeptides in an individual.

The diagnosis may also be achieved by assaying the relative abundance of the 5 36 kDa, 46-43 kDa and 52-56 kDa polypeptides.

Preferably, the relevant polypeptides are detected in an stratum comeum of an individual, whether in vivo or ex-vivo (e.g., in the form of a tape strip).

### Desmoglein I Proteolysis Profile

Similar results are obtained with desmoglein I (DG I). In the stratum corneum of normal skin the most abundant forms of DG I are 95 and 80 kDa which are the proteolytic products of the 160 kDa form. In the stratum corneum of eczema skin there is an increase of 95 and 80 kDa forms, suggesting that the proteolysis process of DG I is enhanced in eczematous skin.

We therefore disclose the diagnosis of a Group I disease or susceptibility to a Group I disease (preferably eczema or susceptibility to eczema) in an individual, by detecting any of the changes described above, preferably the presence of or a modulated level, preferably a higher level of one or more of 95 and 80 kDa polypeptides in an individual. Such diagnosis may also be done by detecting the absence of or a modulated level, preferably a lower level of a 160 kDa desmoglein I polypeptide in an individual.

The diagnosis may also be achieved by assaying the relative abundance of the 80 kDa, 95 kDa and 160 kDa polypeptides. The diagnosis may be done by detecting presence of proteolysis of a 160kDa desmoglein I polypeptide in an individual

### Desmoglein 3 Proteolysis Profile

Desmoglein 3 (Dsg 3) shows in increase in proteolysis. The bands corresponding to the two proteolysis fragments 55 kDa and 100 kDa are strong in the stratum corneum of eczema skin compared to the stratum corneum of normal skin. Both fragments react with the same antibody directed against the cytoplasmic domain. Another band of 80 kDa which appears as a proteolysis product of Dsg 3 is weaker in normal compared to eczematous stratum corneum.

We therefore disclose the diagnosis of a Group I disease or susceptibility to a Group I disease (preferably eczema or susceptibility to eczema) in an individual, by detecting any of the changes described above, preferably the presence of or a modulated level, preferably a higher level of any one, pair or all of a 55 kDa desmoglein 3 polypeptide, an 80 kDa desmoglein 3 polypeptide and a 100 kDa desmoglein 3 polypeptide in an individual.

Preferably, the relevant polypeptides are detected in an stratum corneum of an individual, whether in vivo or ex-vivo (e.g., in the form of a tape strip).

### Plakoglobin Proteolysis Profile

Antibody to plakoglobin revealed three bands 85, 75 and 70 kDa. The 70 and 75 kDa are proteolytic forms of the native protein (85 kDa) which is cleaved by proteases such as SCCE and SCTE during keratinocytes differenciation. The 70 kDa is very weak in eczematic skin (both lesional and non-lesional) and is almost absent in the normal epidermis. 85 and 75 kDa are very strong in eczema skin compared to normal. Eczema and normal skin have two different profiles. The 85 and 75 kDa bands can be use for diagnosis of eczema.

We therefore disclose the diagnosis of a Group I disease or susceptibility to a Group I disease (preferably eczema or susceptibility to eczema) in an individual, by detecting any of the changes described above, preferably the absence of or a modulated level, preferably a lower level of a 70 kDa plakoglobin polypeptide in an individual.

We also disclose the diagnosis of a Group I disease or susceptibility to a Group I disease (preferably eczema or susceptibility to eczema) in an individual,, by detecting the presence of or a modulated level, preferably a higher level of an 85 kDa plakoglobin and/or a 75kDa plakoglobin polypeptide.

The diagnosis may also be achieved by assaying the relative abundance of the 85 kDa, 75 kDa and 70 kDa polypeptides.

Preferably, the relevant polypeptides are detected in an stratum corneum of an individual, whether in vivo or ex-vivo (e.g., in the form of a tape strip).

### Desmocollin 1 Proteolysis Profile

Desmocollin 1 (DGIV/V) is strongly expressed in the suprabasal layers of the normal epidermis.

Antibody to desmoscollin revealed three Dsc1 isoforms in normal epidermis at molecular weigh 70-80; 60-70 and 50-60 kDa. The 50-60 kDa band is strongly expressed in eczema skin including lesional and non-lesional skin, suggesting an increase proteolysis of the desmocollin 1 in eczema skin. The high expression of 50-60 kDa band could be used for proteomic diagnosis of disease with defective skin barrier such as eczema.

We therefore disclose the diagnosis of a Group I disease or susceptibility to a Group I disease (preferably eczema or susceptibility to eczema) in an individual, by detecting any of the changes described above, preferably the presence of or a modulated level, preferably a higher level of a 50-60 kDa Desmocollin 1 polypeptide in an individual.

Preferably, the relevant polypeptides are detected in an stratum corneum of an individual, whether in vivo or ex-vivo (e.g., in the form of a tape strip).

### EXAMPLES D: GENE REGULATION

### Example D5. Expression of Corneodesmosomal Genes in Eczema Assayed Using Oligonucleotide Arrays

The expression of comeodesmosomal genes is assayed as described in eczema patients, using eczema lesional skin ("involved") and eczema non-lesional skin ("uninvolved"). The expression level of each gene in disease (involved and uninvolved) is compared to its expression in normal skin.

Results are shown in Table D5.1 below. Key: ++: normally expressed, +++: strongly expressed; II II: highly expressed, + down-regulation.

**Table D5.1**

| **Gene Name** | **Expression level** | | **GenBank accession number** |
|---|---|---|---|
| | **Involved** | **Uninvolved** | |
| S /comeodesmosin | + | | GB: AF030130 |
| desoplakin | + | + | GB: XM_004463 |
| plakoglobin | + | + | GB: NM 002230; GB:NM_021991 |
| desmoglein 1 | + | + | GB: XM_008810 |
| desmocollin 1 | + | + | GB: MX 008687 |
| envoplakin | + | + | GB: XM_008135;U72543 |
| plectin 1 | + | ++ | GB: NM000445 |
| S100A2 | + | ++ | GB: A1539439;M87068 |
| keratin 6A | ++ | ++ | GB: L42611 |
| keratin 17 | ++ | ++ | GB: Z19574 |
| S100A8 | + | ++ | GB: AI126134 |
| S100A7 | + | +++ | GB: AA586894 |
| S100A9 | + | ++ | GB:W72424 |
| SPRR2A | + | ++ | GB:M21302 |
| SPRR1B | + | + | GB: M19888 |
| SPRK | + | + | GB: A1923984 |
| HCR | + | + | GB: BAA81890 |
| SEEK1 | + | + | GB: BAA88130 |
| SPR1 | + | + | GB: BAB63315 |
| STG | + | ++ | GB: BAA88132 |
| involucrin | + | + | GB: NM_005547 |
| annexin Al /lipocortin | ++ | ++ | GB: X05908 |
| trichohyalin | + | + | GB: NM 005547 |
| collagen, type VI, alpha 3 (COL6A3) | ++ | ++ | GB: NM 004369 |
| loricrin | + | + | GB: XM048902 |

We therefore provide the diagnosis of a Group 1 disease or susceptibility to a Group 1 disease (preferably eczema or susceptibility to eczema) in an individual, by detecting modulation of expression, preferably up-regulation of expression of a polypeptide or nucleic acid selected from the group consisting of keratin 6A (L42611); keratin 17 (Z19574); annexin Al/lipocortin (X05908); and collagen, type VI, alpha 3 (COL6A3) (NM 004369) in an individual.

Furthermore, we provide the diagnosis of a Group 1 disease or susceptibility to a Group 1 disease (preferably eczema or susceptibility to eczema) in an individual, by detecting modulation of expression, preferably down-regulation of expression of a polypeptide or nucleic acid selected from the group consisting of S /comeodesmosin (AF030130); desoplakin (XM_004463); plakoglobin (NM 002230; (NM 021991); desmoglein 1 (XM_008810); desmocollin 1 (MX_008687); envoplakin (XM_008135;U72543); plectin 1 (NM000445); S100A2 (A1539439;M87068); S100A8 (A1126134); S100A7 (AA586894); S100A9); GB:W72424); SPRR2A); GB:M21302); SPRR1B (M19888); SPRK (A1923984); HCR (BAA81890); SEEK1 (BAA88130); SPR1 (BAB63315); STG (BAA88132); involucrin (NM_005547); trichohyalin (NM_005547); and loricrin (XM_048902).

## Claims

1. An ex vivo method for diagnosing, or diagnosing the susceptibility to, a Group I disease associated with a decreased cell-cell adhesion between epithelial cells comprising assaying the profile of epidermally extracted adhesion proteins.

2. A method according to claim 1, wherein the assaying comprises detecting the presence or absence or modulated level of at least one adhesion protein or fragment thereof.

3. A method according to claim 1, wherein the assaying comprises detecting the presence or absence of a polymorphism in an adhesion protein.

4. A method according to claim 1, wherein the assaying comprises assaying the relative abundances of polypeptide isoforms.

5. A method according to any one of the preceding claims wherein the adhesion proteins are corneodesmosomal or desmosomal proteins selected from corneodesmosin (AF030130), desoplakin (XM~004463), plakoglobin (NM~002230) ; (NM~021991), desmoglein 1 (XM-008810), desmocollin 1 (MX008687), envoplakin (XM008135 ; U72543), plectin 1 (NM000445), S100A2 (AI539439 ; M87068), keratin 6A (L42611), keratin 17 (Z19574), S100A8 (AI126134), S100A7 (AA586894), S100A9), GB: W72424), SPRR2A), GB: M21302), SPRR1B (M19888), SPRK (AI923984), HCR (BAA81890), SEEK1 (BAA88130), SPR1 (BAB63315), STG (BAA88132), involucrin (NM005547), annexin A1/lipocortin (X05908), collagen, type VI, alpha 3 (COL6A3) (NM~004369), trichohyalin (NM~005547), and loricrin (XM~048902),
preferably corneodesmosin (also known as S; AF030130), desmoglein 1 (XM~008810), desmocollin 1 (MX~008687), desmoplakins I and II (XM~004463), plakoglobin (also known as PG; NM~002230) and plakophilin (also known as PP),
and more preferably corneodesmosin, desmoglein I, desmoglein 3, plakoglobin, desmoplakin, desmocollin I, envoplakin, a proline-rich protein, preferably a small proline-rich protein (SPRR), SPRR2A, SPRR1 B, SPRK, SPRR2E, SPRR2F, SPRR2B, SPRR2D, SPRR2C, SPRR2G, SPRR1A, SPRR3,SPRR4, involucrin, or loricrin.

6. A method according to any one of the preceding claims wherein the adhesion proteins assayed are a) corneodesmosin, b) desmoglein I, c) desmoglein III, d) plakoglobin and/or e) desmocollin I.

7. A method according to any preceding claim, in which the method comprises detecting any one or more of: (a) relative abundance of the 36 kDa, 46-43 kDa and 52-56 kDa corneodesmosin polypeptides ; (b) the presence of or an elevated level of one or more 36,46-43 kDa corneodesmosin polypeptides; (c) the absence of or a modulated level, preferably a lower level of 52-56 kDa corneodesmosin polypeptides in an individual.

8. A method according to any preceding claim, in which the method comprises detecting any one or more of: (a) relative abundance of the 80 kDa, 95 kDa and 160 kDa desmoglein I polypeptides; the presence of or an elevated level of one or more of 95 and 80 kDa polypeptides; (c) a reduced level of a 160 kDa desmoglein I polypeptide; (d) proteolysis of a 160kDa desmoglein I polypeptide in an individual.

9. A method according to any preceding claim, in which the method comprises detecting the presence of or an elevated level of any one or more of a 55 kDa desmoglein 3 polypeptide, an 80 kDa desmoglein 3 polypeptide and a 100 kDa desmoglein 3 polypeptide in an individual.

10. A method according to any preceding claim, in which the method comprises detecting any one or more of: (a) relative abundance of the 85 kDa, 75 kDa and 70 kDa plakoglobin polypeptides; (b) the absence of or a reduced level of a 70 kDa plakoglobin polypeptide; (c) the presence of or an elevated level of an 85 kDa plakoglobin and/or a 75kDa plakoglobin polypeptide in an individual.

11. A method according to any preceding claim, in which the method comprises detecting any one or more of: (a) relative abundance of the70-80 kDa, 60-70 kDa and 50-60 kDa Desmocollin 1 polypeptides; and (b) the presence of or an elevated level of a 50-60 kDa Desmocollin 1 polypeptide in an individual.

12. A method according to any one of the preceding claims wherein the polypeptide or fragment is detected in the form of a skin biopsy or a tape strip.

13. A method according to any one of the preceding claims wherein the adhesion proteins are detected by Western blot.

14. A method according to any one of the preceding claims wherein the adhesion proteins are detected using antibodies specific for the proteins.

15. A method according to any one of the preceding claims wherein the Group I disease is selected from the group consisting of atopic eczema, sebarrhoeic eczema, irritant contact dermatitis, allergic contact dermatitis, lung atopic asthma, post viral asthma, bronchial hyper-reactivity, chronic obstruction pulmonary disease, Crohn's disease, ulcerative colitis, coeliac disease, peptic ulceration, impetigo, viral warts, Molluscum Contagiosum, bacterial meningitis, viral meningitis, peptic ulceration associated with penetration of Helicobacteria pylori, skin melanoma, squamous cell carcinoma, basal cell carcinoma, cutaneous lymphoma, a skin cancer, a malignancy of gastrointestinal tract and a malignancy of the lung,
and preferably the disease is selected from the group consisting of:
atopic eczema, sebarrhoeic eczema, irritant contact dermatitis, allergic contact dermatitis and impetigo.
